# EUROPEAN PATENT SPECIFICATION

(11) **EP 2 919 795 B1**
(45) Date of publication and mention of the grant of the patent: **30.06.2021**
(21) Application number: 13854825.0
(22) Date of filing: 31.08.2013
(51) Int. Cl.: A61K 35/34, A61L 27/36

(54) **SOLUBILIZATION OF ANTIGEN COMPONENTS FOR REMOVAL FROM TISSUES**
SOLUBILISIERUNG VON ANTIGENKOMPONENTEN ZUR ENTFERNUNG AUS GEWEBE
SOLUBILISATION DE CONSTITUANTS ANTIGÈNES POUR L'ÉLIMINATION À PARTIR DE TISSUS

(30) Priority: 18.11.2012 US 201261727738 P
(43) Date of publication of application: 23.09.2015
(60) Divisional application: 21174324.0
(73) Proprietor: The Regents of the University of California, Oakland, CA 94607 (US)
(72) Inventor: GRIFFITHS, Leigh G., Davis, California 95618 (US); PAPALAMPROU, Angeliki, Davis, California 95618 (US); WONG, Maelene L., Woodland, California 95776 (US)
(74) Representative: J A Kemp LLP
(86) International application number: PCT/US2013/057741
(87) International publication number: WO 2014/077934

(56) References cited:
- WO-A2-2011/132089
- US-A1- 2011 008 397
- GRIFFITHS L G ET AL: "Immunoproteomic identification of bovine pericardium xenoantigens", BIOMATERIALS, ELSEVIER SCIENCE PUBLISHERS BV., BARKING, GB, vol. 29, no. 26, 1 September 2008 (2008-09-01), pages 3514-3520, XP022822733, ISSN: 0142-9612, DOI: 10.1016/J.BIOMATERIALS.2008.05.006 [retrieved on 2008-06-02]
- FILATOV ET AL: 'DIRECT AND INDIRECT ANTIBODY-INDUCED TX-100 RESISTANCE OF CELL SURFACE ANTIGENS' IMMUNOLOGY LETTERS vol. 85, 2003, pages 287 - 295, XP055248793
- WONG ET AL: 'THE ROLE OF PROTEIN SILUBILIZATION IN ANTIGEN REMOVAL FROM XENOGENEIC TISSUE FORHEART VALVE TISSUE ENGINEERING' BIOMATERIALS vol. 32, 31 July 2011, pages 8129 - 8138, XP028277123
- NAKASHIMA ET AL: 'COMPARISON OF STRUCTURE AND FUNCTION OF HUMAN ERYTHROCYTE AND HUMAN MUSCLE ACTIN' PORC.NATL.ACAD.SCI vol. 76, no. 2, February 1979, pages 935 - 938, XP055248796
- SPUDICH ET AL: 'THE REGULATION OF RABBIT SKELETAL MUSCLE CONTRACTION' J.BIOL.CHEM. vol. 246, 1971, pages 4866 - 4871, XP055248800
- None

## Description

### FIELD

The present invention relates to methods for removing antigens from intact tissues by sequentially destabilizing and/or depolymerizing cytoskeletal components and removing and/or reducing water-soluble antigens and lipid-soluble antigens. The disclosure further relates to tissue scaffolding and decellularized extracellular matrix produced by such methods.

### BACKGROUND

Previously described myocardial tissue decellularization methods have been solely based on the ubiquitous use of harsh denaturing detergents, mainly SDS, in concentrations as high as 1% in hypotonic water solutions. Although effective in solubilizing cellular and tissue components, it has been shown that these methods are not successful in removing antigenic determinants, while they are often detrimental to the extracellular matrix (removing elastin, glycosaminoglycans and damaging collagen structure). Further, commonly utilized detergents are toxic to repopulating cells reducing the chances of successful recellularization strategies for the produced scaffolds.

Previously reported methods to produce a myocardial scaffold have been based on detergent-based decellularization methods. More specifically, these approaches included the use of one detergent (SDS, Triton-X100, Saponin) with protease inhibitors (to prevent extracellular matrix protein degradation) and occasionally enzymatic treatments (such as trypsin) and nucleases for nucleic acid degradation in different concentrations and combinations. All these reports determine loss of nuclei and cellular components and production of an acellular scaffold as their outcome measure for protocol success. As we have already shown in our laboratory this assumption is not valid, since antigens may be associated with non-cellular components of the tissue. Additionally, the omission of a reducing agent in all these treatments would be expected to result in protein precipitation rather than solubilization and extraction, regardless of the concentration of the detergent used.

Griffiths et al., Biomaterials, 2008, 29(26), 3514-3520, describes immunoproteomic identification of bovine pericardium xenoantigens. WO 2011/132089 relates to methods for producing tissue decellularized, sterilized and bioprosthetic tissues. Filatov et al., Immunology Letters, 2003, vol. 85, pages 287 - 295, describes direct and indirect antibody-induced TX-100 resistance of cell surface antigens. US2011/008397 relates to compartmental extract compositions for tissue engineering. Wong et al., Biomaterials, 2011, vol. 32, pages 8129 - 8138, describes the role of protein solubilization in antigen removal from xenogenic tissue for heart valve tissue engineering. Nakashima et al., Proc. Natl. Acad. Sci, 1979, vol. 76, no. 2, pages 935 - 938, relates to the comparison of structure and function of human erythrocyte and human muscle actin. Spudich et al., J. Biol. Chem., 1971, vol. 246, pages 4866 - 4871, describes the regulation of rabbit skeletal muscle contraction.

### SUMMARY

The present invention relates to methods for removing antigen components from tissue, *e.g.,* to create a tissue scaffolding and/or a substantially decellularized extracellular matrix, *e.g.,* for use in tissue transplantation, tissue regeneration, and/or model matrices for study of cellular/ECM interactions. In one embodiment, the present invention provides a method of removing immunogenic antigens from an intact tissue, comprising:
a) contacting the intact tissue with one or more actin depolymerization agents, and/or one or more microtubule depolymerization or microtubule destabilizing agents;
b) solubilizing water-soluble antigens in the tissue, wherein the water-soluble antigens are solubilized in a first solution comprising a first buffering agent, a reducing agent, a protease inhibitor, and one or more metal halide salts, wherein the first solution does not comprise an amphiphile;
c) separating the tissue from the solubilized water-soluble antigens;
d) solubilizing lipid-soluble antigens in the tissue, wherein the lipid-soluble antigens are solubilized in a second solution comprising a second buffering agent, a reducing agent, a protease inhibitor, one or more salts suitable for maintaining protein solubility and an amphiphile; and
e) separating the tissue from the solubilized lipid-soluble antigens; thereby removing immunogenic antigens from the tissue, wherein the method yields a substantially intact extracellular matrix (ECM) compatible with viable cell repopulation. In various embodiments, the water-soluble antigen components are solubilized and removed from the tissue, and then the lipid-soluble antigen components are subsequently solubilized and removed from the tissue. In various embodiments, the lipid-soluble antigen components are solubilized and removed from the tissue, and then the water-soluble antigen components are subsequently solubilized and removed from the tissue. As appropriate, the tissue may be a part of an organ or an intact organ. The methods are performed *in vitro.*

In varying embodiments, the steps of the method are performed in the order set forth above. In varying embodiments, one or more of the steps are repeated, *e.g.,* one, two, three or more times, as appropriate or desired.

In a related aspect, the invention provides methods as set forth above for removing immunogenic antigens from a muscle tissue, comprising:
a) relaxing the muscle tissue in a relaxing solution comprising a chemical phosphatase and/or myosin ATPase inhibitor and/or a myosin light chain kinase (MLCK) inhibitor and/or an energy source molecule;
b) contacting the muscle tissue with one or more cytoskeletal destabilizing and/or depolymerizing agents;
c) contacting the muscle tissue with a concentrated salt solution;
d) solubilizing water-soluble antigens in the tissue as set forth above;
e) separating the muscle tissue from the solubilized water-soluble antigens;
f) solubilizing lipid-soluble antigens in the tissue; and
g) separating the muscle tissue from the solubilized lipid-soluble antigens; thereby removing immunogenic antigens from the muscle tissue. In various embodiments, a wash or rinse step is performed after step a), separating the tissue from the relaxing solution. In various embodiments, a wash or rinse step is performed after step b), separating the tissue from the one or more cytoskeletal destabilizing agents and destabilized and/or depolymerized cytoskeletal proteins. In various embodiments, a wash or rinse step is performed after step c), separating the tissue from the concentrated salt solution and solubilized sarcomeric components. In varying embodiments, the steps of the method are performed in the order set forth above. In varying embodiments, the steps for removing immunogenic antigens from a muscle tissue are performed in the following order: a), b), c), d), e), f) and g). In varying embodiments, the steps for removing immunogenic antigens from a muscle tissue are performed in the following order: a), f), g), b), c), d) and e). In varying embodiments, the steps for removing immunogenic antigens from a muscle tissue are performed in the following order: a), d), e), b), c), f) and g). In varying embodiments, the steps for removing immunogenic antigens from a muscle tissue are performed in the following order: a), d), e), f), g), b) and c). In varying embodiments, one or more of the steps are repeated, *e.g.,* one, two, three or more times, as appropriate or desired. For example, in some embodiments, steps b), c), d) and e) are repeated one or more times. In varying embodiments, the steps for removing immunogenic antigens from a muscle tissue are performed in the following order: a), b), c), d), e), f), g), b), c), and e). In varying embodiments, the steps for removing immunogenic antigens from a muscle tissue are performed in the following order: a), f), g), b), c), d), e), f), and g).

In varying embodiments of the methods, the lipid-soluble antigens are solubilized and separated first, and then the water-soluble antigens are solubilized and separated. In varying embodiments, the water-soluble antigens are solubilized and separated first, and then the lipid-soluble antigens are solubilized and separated. In varying embodiments, the tissue is contacted with one or more one or more cytoskeletal destabilizing and/or depolymerizing agents concurrently with the solubilization and separation of water soluble antigens.

With respect to further embodiments of the methods, in some embodiments, the immunogenic antigens are selected from the group consisting of protein antigens, lipid antigens and carbohydrate antigens. In some embodiments, the method does not comprise contacting the tissue with a detergent selected from the group consisting of sodium dodecyl sulfate, Triton-X-100, Triton-X-114, Triton-X-200 and sodium deoxycholate. In some embodiments, the method does not comprise contacting the tissue with a protease, for example, trypsin.

In some embodiments, the one or more cytoskeletal destabilizing agents comprise one or more actin depolymerization agents. In some embodiments, the one or more actin depolymerization agents are selected from the group consisting of Cytochalasin A, Cytochalasin B, Cytochalasin C, Cytochalasin D, Cytochalasin E, Cytochalasin F, Cytochalasin G, Cytochalasin H, Cytochalasin I, Cytochalasin J, Latrunculin A, Latrunculin B, Swinholide A, Misakinolide A, Bistheonelide A, Scytophycin A, Scytophycin B, Scytophycin D, Scytophycin E, 19-0-Demethylscytophycin C, 6-Hydroxyscytophycin B, 6-Hydroxy-7-o-methylscytophycin E and tolytoxin, Mycalolide A, Mycalolide B, Mycalolide C, secomycalolide A and 30-hydroxymycalolide A, Halichondramide, (19Z)-halichondramide, kabiramides B, kabiramides C, kabiramides D, kabiramides G, kabiramides J, kabiramides K, ulapualide A, jaspamide, Dihydrohalichondramide, Aplyronine A, Aplyronine B, Aplyronine C, Pectenotoxin 2, Pectenotoxin 6, and Migrastatin. In some embodiments, the actin depolymerization agents depolymerize filamentous cytoskeletal actin (F-actin). In some embodiments, the actin depolymerization agents depolymerize filamentous α-sarcomeric actin (F-actin).

In some embodiments, the one or more cytoskeletal destabilizing agents comprise one or more microtubule depolymerization or destabilizing agents. In some embodiments, the one or more microtubule depolymerization or destabilizing agents is selected from the group consisting of colchicine, colcemid, vinblastine, vincristine, myoseverin, nocodazole, podophyllotoxin, polygamain and taxol.

The water-soluble antigens are solubilized in a solution comprising a buffering agent, a reducing agent, a protease inhibitor, and one or more salts suitable for maintaining protein solubility. In some embodiments, the buffering agent maintains a pH in the range of about 4-11, *e.g.,* a pH in the range of about 4-6, 8-11, 5-10 or 6-9. In some embodiments, the buffering agent maintains a pH of at least about 8. In some embodiments, the one or more salts comprise a monovalent or a divalent anion. In some embodiments, the one or more salts comprise a metal halide salt. In some embodiments, the metal halide salt is selected from the group consisting of LiF, LiCl, LiBr, LiI, NaF, NaCl, NaBr, NaI, KF, KCl, KBr, KI, RbF, RbCl, RbBr, RbI, CsF, CsCl, CsBr, CsI, BeF₂, BeCl₂, BeBr₂, BeI₂, MgF₂, MgCl₂, MgBr₂, MgI₂, CaF₂, CaCl₂, CaBr₂, CaI₂, SrF₂, SrCl₂, SrBr₂, SrI₂, BaF₂, BaCl₂, BaBr₂, BaI₂, and mixtures thereof. In some embodiments, the reducing agent is selected from the group consisting of Tributylphosphine (TBP), beta mercaptoethanol, 2-mercaptoethylamine, tris(2-carboxyethyl)phosphine (TCEP), cysteine-HCl, and dithiothreitol (DTT). In some embodiments, the water-soluble antigens are solubilized in a solution that comprises one or more of an antibacterial agent and/or an antifungal agent. In some embodiments, the water-soluble antigens are solubilized in a solution that comprises a chelation agent. In some embodiments, the chelation agent is selected from the group consisting of ethylenediaminetetraacetic acid (EDTA), diethylenetriaminepentaacetic acid (DTPA), Citric Acid, N,N-bis(carboxymethyl)glycine (NTA), and the meso isomer of Dimercaptosuccinic acid (DMSA). In some embodiments, the water-soluble antigens are solubilized in a solution that does not comprise an amphiphile. In some embodiments, the water-soluble antigens are solubilized in a solution that does not comprise a detergent. In some embodiments, the water-soluble antigens are solubilized in a solution that comprises a non-detergent sulfobetaine. In some embodiments, the water-soluble antigens are solubilized in a solution comprising Tris-HCl, dithiothreitol (DTT), a protease inhibitor, and KCl.

The lipid-soluble antigens are solubilized in a solution comprising a buffering agent, a reducing agent, a protease inhibitor, one or more salts suitable for maintaining protein solubility and an amphiphile. In some embodiments, the amphiphile is a zwitterionic detergent. In some embodiments, the amphiphile is a sulfobetaine. In some embodiments, the sulfobetaine is selected from the group consisting of 3-[N,N-Dimethyl(3-myristoylaminopropyl)ammonio]propanesulfonate (amidosulfobetaine-14; ASB-14); amidosulfobetaine-16 (ASB-16); 4-n-Octylbenzoylamido-propyl-dimethylammonio sulfobetaine (ASB-C8Ø); 3-(N,N-Dimethyloctylammonio) propanesulfonate inner salt (SB3-8); N-Decyl-N,N-dimethyl-3-ammonio-1-propanesulfonate (SB 3-10), N-Decyl-N,N-dimethyl-3-ammonio-1-propanesulfonate (SB 3-12), N-Tetradecyl-N,N-dimethyl-3-ammonio-1-propanesulfonate (SB 3-14); 3-(N,N-Dimethylpalmitylammonio) propanesulfonate (SB3-16); 3-(N,N-Dimethyloctadecylammonio) propanesulfonate (SB3-18); 3-(1-Pyridinio)-1-propanesulfonate (NDSB-201); 3-(Benzyldimethylammonio) propanesulfonate (NDSB-256); NDSB-211, NDSB-195, NDSB-221; 3-[(3-Cholamidopropyl)dimethylammonio]-1-propanesulfonate (CHAPS), 3-[(3-Cholamidopropyl)dimethylammonio]-2-hydroxy-1-propanesulfonate (CHAPSO), and mixtures thereof. In some embodiments, the lipid-soluble antigens are solubilized in a solution comprising Tris-HCl, dithiothreitol (DTT), a protease inhibitor, KCl and ASB-14.

In some embodiments, the method yields a substantially intact extracellular matrix (ECM) compatible with viable cell repopulation. In some embodiments, the methods further comprise repopulating the ECM with live cells. In some embodiments, the live cells are autologous, allogeneic or xenogeneic to the ECM. In some embodiments, the live cells comprise mesenchymal stem cells. In some embodiments, the live cells comprise cells of the same tissue type as the tissue from which the antigens are removed.

In some embodiments, the tissue is epithelial tissue, endothelial tissue, muscle tissue, or connective tissue. In some embodiments, the tissue is selected from the group consisting of cardiac muscle tissue, striated or skeletal muscle tissue, or smooth muscle tissue, heart, pericardium, heart valve, vessel, vascular conduit, artery, vein, skin, dermis, pericardium, dura, intestinal submucosa, ligament, tendon, bone, cartilage, ureter, urinary bladder, kidney, skin, lung, liver, and umbilical cord. In some embodiments, the tissue is an intact tissue. In some embodiments, the tissue is within or a part of an intact organ. In some embodiments, at least about 80%, for example, at least about 85%, 90%, 93%, 95%, 97%, 99%, or more, of the water soluble antigens are removed from the tissue. In some embodiments, at least about 60%, for example, at least about 65%, 70%, 75%, 80%, 85%, 90%, 93%, 95%, 97%, 99%, or more, of the lipid soluble antigens are removed from the tissue.

In some embodiments, the tissue is muscle tissue. In some embodiments, the muscle tissue is cardiac muscle tissue, striated or skeletal muscle tissue, or smooth muscle tissue. In some embodiments, the method comprises the step of relaxing the muscle tissue prior to contacting the tissue with one or more cytoskeletal destabilizing agents. In some embodiments, the muscle tissue is relaxed in a relaxing solution comprising a chemical phosphatase and/or myosin ATPase inhibitor, *e.g.,* 2,3-Butanedione monoxime (BDM), blebbisatin, vanadate ions, and/or sodium orthovanadate. In some embodiments, the muscle tissue is relaxed in a relaxing solution comprising a myosin light chain kinase (MLCK) inhibitor, *e.g.,* N-(2-Aminoethyl)-5-chloronaphthalene-1-sulfonamide-HCl, ML-7, ML-9, K252a, K252b, Go7874-HCl, myosin light chain kinase inhibitor peptide 18, piceatannol, and/or staurosporine. In some embodiments, the muscle tissue is relaxed in a relaxing solution comprising an energy source molecule. In some embodiments, the energy source molecule is selected from the group consisting of a nucleotide 5'-triphosphate (NTP), adenosine, inosine, aspartate, glutamate, creatine phosphate, a Kreb's cycle precursor or intermediate, glucose, and dextrose. In some embodiments, the energy source molecule is a nucleotide 5'-triphosphate (NTP) selected from the group consisting of adenosine 5'-triphosphate (ATP), inosine 5'-triphosphate (ITP), guanidine 5'-triphosphate (GTP), cytidine 5'-triphosphate (CTP), and uridine 5'-triphosphate (UTP). In some embodiments, the energy source molecule is a precursor of adenosine 5'-triphosphate (ATP). In some embodiments, the energy source molecule is adenosine 5'-triphosphate (ATP). In some embodiments, the relaxing solution further comprises a calcium ion chelating agent. In some embodiments, the relaxing solution further comprises a permeabilization agent. In some embodiments, the muscle tissue is contacted with a concentrated salt solution. In some embodiments, the concentrated salt solution comprises one or more salts in a concentration range from about 0.5 M to about 3.0 M, *e.g.,* about 0.6 M, 0.7 M, 0.8 M, 0.9 M, 1.0 M, 1.1 M, 1.2 M, 1.3 M, 1.4 M, 1.5 M, 1.6 M, 1.7 M, 1.8 M, 1.9 M, 2.0 M, 2.1 M, 2.2 M, 2.3 M, 2.4 M, 2.5 M, 2.6 M, 2.7 M, 2.8 M, 2.9 M, or 3.0 M. In some embodiments, the concentrated salt solution comprises one or more metal halide salts. In some embodiments, the metal halide salt is selected from the group consisting of LiF, LiCl, LiBr, LiI, NaF, NaCl, NaBr, NaI, KF, KCl, KBr, KI, RbF, RbCl, RbBr, RbI, CsF, CsCl, CsBr, CsI, BeF₂, BeCl₂, BeBr₂, BeI₂, MgF₂, MgCl₂, MgBr₂, MgI₂, CaF₂, CaCl₂, CaBr₂, CaI₂, SrF₂, SrCl₂, SrBr₂, SrI₂, BaF₂, BaCl₂, BaBr₂, BaI₂, and mixtures thereof. In some embodiments, the concentrated salt solution comprises KCl and KI. In some embodiments, the concentrated salt solution comprises 0.6 M KCl and 1.0 M KI. In some embodiments, sarcomeric constituents are not detectable in the muscle tissue. In some embodiments, at least about 90% of the sarcomeric constituents are removed, *e.g.,* at least about 93%, 95%, 97%, 98%, 99% or all (100%) sarcomeric constituents are removed.

In a further aspect, the disclosure provides tissue scaffolds produced by the methods described above and herein. In another aspect, the disclosure provides kits comprising a tissue scaffold produced by the methods described above and herein. In a related aspect, the disclosure provides decellularized extracellular matrix (ECM) produced by the methods described above and herein. In varying aspects, the ECM and/or tissue scaffolds are free of residual sodium dodecyl sulfate. In varying aspects, the ECM and/or tissue scaffolds induce little or no immune response in a host that is xenogeneic or allogeneic to the ECM. In some aspects, the ECM and/or tissue scaffolds do not comprise detectable sarcomeric consituents. In varying aspects, the ECM comprises ECM structure, ECM biochemical composition and ECM tensile strength that is substantially the same as the ECM prior to decellularization, wherein the ECM is compatible with viable cell repopulation, and is substantially free of endogenous antigens.

In a further aspect, the disclosure provides kits comprising (i) a solution for destabilizing cytoskeletal polymers, (ii) a solution for solubilizing water soluble antigens and (iii) a solution for solubilizing lipid soluble antigens.

With respect to aspects of the kits, in some aspects, the solution for destabilizing cytoskeletal polymers comprises one or more actin depolymerization agents. In some aspects, the one or more actin depolymerization agents are selected from the group consisting of Cytochalasin A, Cytochalasin B, Cytochalasin C, Cytochalasin D, Cytochalasin E, Cytochalasin F, Cytochalasin G, Cytochalasin H, Cytochalasin I, Cytochalasin J, Latrunculin A, Latrunculin B, Swinholide A, Misakinolide A, Bistheonelide A, Scytophycin A, Scytophycin B, Scytophycin D, Scytophycin E, 19-0-Demethylscytophycin C, 6-Hydroxyscytophycin B, 6-Hydroxy-7-o-methylscytophycin E and tolytoxin, Mycalolide A, Mycalolide B, Mycalolide C, secomycalolide A and 30-hydroxymycalolide A, Halichondramide, (19Z)-halichondramide, kabiramides B, kabiramides C, kabiramides D, kabiramides G, kabiramides J, kabiramides K, ulapualide A, jaspamide, Dihydrohalichondramide, Aplyronine A, Aplyronine B, Aplyronine C, Pectenotoxin 2, Pectenotoxin 6, and Migrastatin. In some aspects, the actin depolymerization agents depolymerize filamentous cytoskeletal actin. In some aspects, the actin depolymerization agents depolymerize α-sarcomeric actin (F-actin). In some aspects, the solution for destabilizing cytoskeletal polymers comprises one or more microtubule depolymerization or destabilizing agents. In some aspects, the one or more microtubule depolymerization or destabilizing agents is selected from the group consisting of colchicine, colcemid, vinblastine, vincristine, myoseverin, nocodazole, podophyllotoxin, polygamain and taxol.

In some aspects, the solution for solubilizing water soluble antigens comprises a buffering agent, a reducing agent, a protease inhibitor, and one or more salts suitable for maintaining protein solubility. In some aspects, the buffering agent maintains a pH in the range of about 4-11, *e.g.,* a pH in the range of about 4-6, 8-11, 5-10 or 6-9. In some aspects, the buffering agent maintains a pH of at least about 8. In some aspects, the one or more salts comprise a monovalent or a divalent anion. In some aspects, the one or more salts comprise a metal halide salt. In some aspects, the metal halide salt is selected from the group consisting of LiF, LiCl, LiBr, LiI, NaF, NaCl, NaBr, NaI, KF, KCl, KBr, KI, RbF, RbCl, RbBr, RbI, CsF, CsCl, CsBr, CsI, BeF₂, BeCl₂, BeBr₂, BeI₂, MgF₂, MgCl₂, MgBr₂, MgI₂, CaF₂, CaCl₂, CaBr₂, CaI₂, SrF₂, SrCl₂, SrBr₂, SrI₂, BaF₂, BaCh, BaBr₂, BaI₂, and mixtures thereof. In some aspects, the reducing agent is selected from the group consisting of Tributylphosphine (TBP), beta mercaptoethanol, 2-mercaptoethylamine, tris(2-carboxyethyl)phosphine (TCEP), cysteine-HCl, and dithiothreitol (DTT). In some aspects, the solution for solubilizing water soluble antigens comprises one or more of an antibacterial agent and/or an antifungal agent. In some aspects, the solution for solubilizing water soluble antigens comprises a chelation agent. In some aspects, the chelation agent is selected from the group consisting of ethylenediaminetetraacetic acid (EDTA), diethylenetriaminepentaacetic acid (DTPA), Citric Acid, N,N-bis(carboxymethyl)glycine (NTA), and the meso isomer of Dimercaptosuccinic acid (DMSA). In some aspects, the solution for solubilizing water soluble antigens does not comprise an amphiphile. In some aspects, the solution for solubilizing water soluble antigens does not comprise a detergent. In some aspects, the solution for solubilizing water soluble antigens comprises a non-detergent sulfobetaine. In some aspects, the solution for solubilizing water soluble antigens comprises Tris-HCl, dithiothreitol (DTT), a protease inhibitor, and KCl.

In some aspects, the solution for solubilizing lipid soluble antigens comprises a buffering agent for maintaining pH of at least 8.0, a reducing agent, a protease inhibitor, one or more salts suitable for maintaining protein solubility and an amphiphile. In some aspects, the amphiphile is a zwitterionic detergent. In some aspects, the amphiphile is a sulfobetaine. In some aspects, the sulfobetaine is selected from the group consisting of 3-[N,N-Dimethyl(3-myristoylaminopropyl)ammonio]propanesulfonate (amidosulfobetaine-14; ASB-14); amidosulfobetaine-16 (ASB-16); 4-n-Octylbenzoylamido-propyl-dimethylammonio sulfobetaine (ASB-C8Ø); 3-(N,N-Dimethyloctylammonio) propanesulfonate inner salt (SB3-8); N-Decyl-N,N-dimethyl-3-ammonio-1-propanesulfonate (SB 3-10), N-Decyl-N,N-dimethyl-3-ammonio-1-propanesulfonate (SB 3-12), N-Tetradecyl-N,N-dimethyl-3-ammonio-1-propanesulfonate (SB 3-14); 3-(N,N-Dimethylpalmitylammonio) propanesulfonate (SB3-16); 3-(N,N-Dimethyloctadecylammonio) propanesulfonate (SB3-18); 3-(1-Pyridinio)-1-propanesulfonate (NDSB-201); 3-(Benzyldimethylammonio) propanesulfonate (NDSB-256); NDSB-211, NDSB-195, NDSB-221; 3-[(3-Cholamidopropyl)dimethylammonio]-1-propanesulfonate (CHAPS), 3-[(3-Cholamidopropyl)dimethylammonio]-2-hydroxy-1-propanesulfonate (CHAPSO), and mixtures thereof. In some aspects, the lipid-soluble antigens are solubilized in a solution comprising Tris-HCl, dithiothreitol (DTT), a protease inhibitor, KCl and ASB-14.

In some aspects, the kits further comprise (iv) a relaxing solution comprising an energy source molecule and/or a chemical phosphatase and/or myosin ATPase inhibitor and/or myosin light chain kinase (MLCK) inhibitor. In varying aspects, the chemical phosphatase and/or myosin ATPase inhibitor is selected from 2,3-Butanedione monoxime (BDM), blebbisatin, vanadate ions, sodium orthovanadate. In varying aspects, the chemical phosphatase and/or myosin ATPase inhibitor is 2,3-Butanedione monoxime (BDM). In some aspects, the myosin light chain kinase (MLCK) inhibitor is selected from N-(2-Aminoethyl)-5-chloronaphthalene-1-sulfonamide-HCl, ML-7, ML-9, K252a, K252b, Go7874-HCl, myosin light chain kinase inhibitor peptide 18, piceatannol, and/or staurosporine. In some aspects, the energy source molecule is selected from the group consisting of a nucleotide 5'-triphosphate (NTP), adenosine, inosine, aspartate, glutamate, creatine phosphate, a Kreb's cycle precursor or intermediate, glucose, and dextrose. In some aspects, the energy source molecule is a nucleotide 5'-triphosphate (NTP) selected from the group consisting of adenosine 5'-triphosphate (ATP), inosine 5'-triphosphate (ITP), guanidine 5'-triphosphate (GTP), cytidine 5'-triphosphate (CTP), and uridine 5'-triphosphate (UTP). In some aspects, the energy source molecule is a precursor of adenosine 5'-triphosphate (ATP). In some aspects, the energy source molecule is adenosine 5'-triphosphate (ATP). In some aspects, the relaxing solution further comprises a calcium ion chelating agent. In some aspects, the energy source molecule is a precursor of adenosine 5'-triphosphate (ATP). In some aspects, the relaxing solution further comprises a permeabilization agent.

In some aspects, the kits further comprise (v) a concentrated salt solution. In some aspects, the concentrated salt solution comprises one or more salts in a concentration range from about 0.5 M to about 3.0 M, *e.g.,* about 0.6 M, 0.7 M, 0.8 M, 0.9 M, 1.0 M, 1.1 M, 1.2 M, 1.3 M, 1.4 M, 1.5 M, 1.6 M, 1.7 M, 1.8 M, 1.9 M, 2.0 M, 2.1 M, 2.2 M, 2.3 M, 2.4 M, 2.5 M, 2.6 M, 2.7 M, 2.8 M, 2.9 M, or 3.0 M. In some aspects, the concentrated salt solution comprises one or more metal halide salts. In some aspects, the metal halide salt is selected from the group consisting of LiF, LiCl, LiBr, LiI, NaF, NaCl, NaBr, NaI, KF, KCl, KBr, KI, RbF, RbCl, RbBr, RbI, CsF, CsCl, CsBr, CsI, BeF₂, BeCl₂, BeBr₂, BeI₂, MgF₂, MgCl₂, MgBr₂, MgI₂, CaF₂, CaCl₂, CaBr₂, CaI₂, SrF₂, SrCl₂, SrBr₂, SrI₂, BaF₂, BaCl₂, BaBr₂, BaI₂, and mixtures thereof. In some aspects, the concentrated salt solution comprises KCl and KI. In some aspects, the concentrated salt solution comprises 0.6 M KCl and 1.0 M KI.

### DEFINITIONS

The terms "tissue" or "biological tissue" interchangeably refer to a collection of interconnected cells and extracellular matrix that perform a similar function or functions within an organism. Biological tissues include, without limitation, connective tissue, muscle tissue, nervous tissue (of the brain, spinal cord, and nerves), epithelial tissue, and organ tissue. Connective tissue includes fibrous tissue, *e.g.,* fascia, tendon, ligaments, heart valves, bone, and cartilage. Muscle tissue includes skeletal muscle tissue, smooth muscle tissue, *e.g.,* esophageal, stomach, intestinal, bronchial, uterine, urethral, bladder, and blood vessel tissue, and cardiac muscle tissue. Epithelial tissue includes simple epithelial tissue, *e.g.,* alveolar epithelial tissue, blood vessel endothelial tissue, and heart mesothelial tissue, and stratified epithelial tissue. The biological tissue can additionally be selected, without limitation, from the group consisting of heart valve, vessel, vascular conduit, artery, vein, skin, dermis, pericardium, dura, intestinal submucosa, ligament, tendon, bone, cartilage, ureter, urinary bladder, liver, lung, umbilical cord, and heart. Multiple tissues/tissue types comprise organs. Organs are included herein under the terms "tissue and/or "biological tissue."

The phase "intact tissue" refers to tissue that has not been minced or homogenized. The intact tissue may be a whole tissue or a complete organ.

The term "organ" as used herein refers to a collection of tissues joined in a structural unit to serve a common function.

The term "dermis" as used herein refers to the layer of skin between the epidermis and the subcutaneous tissues.

The term "epithelial tissue" as used herein refers to the tissue covering the whole surface of the body or lining certain organ systems exposed to the external environment, such as the gastrointestinal tract, the urogenital tract, or the lung. It is made up of cells closely packed and arranged in at least one layer. This tissue is specialized to form a covering or lining of all internal and external body surfaces.

The terms "patient," "subject" or "individual" interchangeably refers to a mammal, for example, a human or a non-human mammal, including primates (e.g., macaque, pan troglodyte, pongo), a domesticated mammal (e.g., felines, canines), an agricultural mammal (e.g., bovine, ovine, porcine, equine) and a laboratory mammal or rodent (e.g., rattus, murine, lagomorpha, hamster).

The term "cellular and/or soluble macromolecular component" as used herein refers to soluble substances constituting portions of the cell or produced by cells, including cell membranes, cytosol, and soluble macromolecules (e.g. proteins, nucleic acids, polypeptides, glycoproteins, carbohydrates, lipids, phospholipids, etc.). Cellular and/or soluble macromolecular components that induce an immune response in a subject are immunogenic antigens.

The term "recellularization" as used herein refers to removing the cells from a biological tissue, for example, an organ, leaving only the extracellular matrix to be subsequently repopulated with cells, preferably live cells. Recellularization is especially useful in tissue engineering.

The term "extracellular matrix" (ECM) as used herein refers to the extensive and complex structure between the cells--the extracellular part of the biological tissue. The ECM generally comprises the structural component of the tissue, including its organization, shape, and strength (*i.e.,* ability to resist external forces). Due to its diverse nature and composition, the ECM can serve many additional functions, such as providing support and anchorage for cells, segregating tissues from one another, and regulating intercellular communication. The ECM can influence a cell's dynamic behavior. In addition, it sequesters a wide range of cellular growth factors and acts as a local depot for them. Included in the ECM are insoluble structural molecules that have been secreted by cells and comprise components such as collagen, elastin, and large soluble proteoglycans.

A "decellularized extracellular matrix" refers to an ECM wherein the endogenous cells have been substantially removed. In various aspects, the decellularized ECM is isolated or separated from at least about 60%, 70%, 80%, 90%, 95%, 99%, or more, of endogenous cellular material. The presence or extent of endogenous cellular material can be determined using any method known in the art, *e.g.,* Western blotting, detection of nuclei, microscopy, *etc.*

The phrase "substantially intact" with respect to a tissue scaffold and/or decellularized extracellular matrix (ECM) refers to an ECM that has been subject to antigen removal and has structural integrity, biochemical composition and tensile strength that is not significantly different from an ECM from the same tissue before it is subject to antigen removal.

The phrase "substantially free of endogenous antigens" with respect to a tissue scaffold and/or decellularized extracellular matrix (ECM) refers to a tissue scaffold and/or ECM wherein the endogenous antigen components (*e.g.,* proteins, lipids, carbohydrates, nucleic acids) have been substantially removed. In various aspects, the decellularized ECM is isolated or separated from at least about 60%, 70%, 80%, 90%, 95%, 99%, or more, of endogenous antigen components. The presence or extent of endogenous antigen components can be determined using any method known in the art, *e.g.,* immunoassays, Western blotting, ELISA, gel electrophoresis, lymphocyte proliferation assays, *etc.* In various aspects, tissue scaffolds and/or ECM that are substantially free of endogenous antigens do not elicit a significant or destructive immune response, *e.g.,* an allogeneic and/or xenogeneic immune response, *in vitro* or *in vivo,* directed against the tissue scaffolds and/or ECM.

The term "sulfobetaine" refers to zwitterionic amphiphilic molecules that contain a polarized sulfobetaine head group (*e.g.,* dimethylsulfonioacetate (CH₃)₂S⁺-CH₂-CO₂⁻). In various embodiments, the head group is followed by a three-carbon linkage between the quaternary ammonium and the amido nitrogen. In various embodiments, the sulfobetaine comprises a linear hydrocarbon tail composed of 13 to 16 carbons. The sulfobetaine can be a detergent or a non-detergent molecule.

### BRIEF DESCRIPTION OF THE DRAWINGS

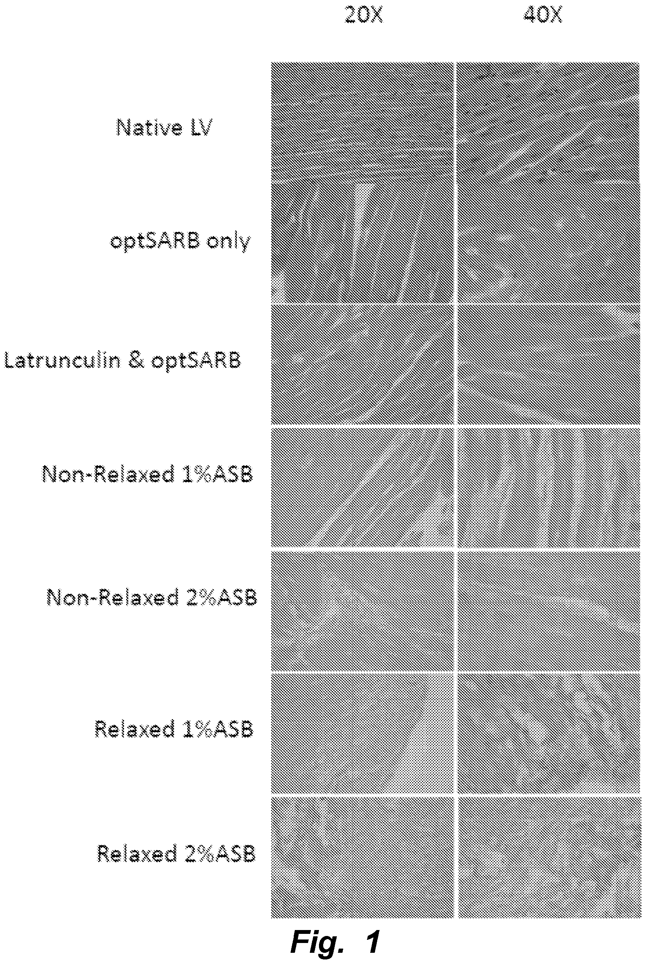

Figure 1 illustrates histologic sections of heart muscle scaffolds generated using the protocols described herein. **Top Row:** Native LV - native unprocessed rat left ventricular (LV) heart muscle tissue. **Second Row:** Following hydrophilic antigen removal alone using solubilization antigen removal buffer (SARB, containing DTT and KCl) for 2 days, cardiomyocytes show evidence of mild cytoplasmic and nuclear vacuolation. **Third Row:** Addition of Latrunculin to SARB protocol results in increased coalescing areas of cytoplasmic vacuolation, mild disruption of sarcomeric structure and almost complete removal of nuclei. **Fourth and Fifth Rows:** Addition of ASB-14 (hydrophobic solubilization) prior to application of Latrunculin/SARB results in complete removal of some cardiomyocytes while retaining normal structure of the extracellular matrix (ECM) in these regions, although some cardiomyocytes remain. Sixth Row: Relaxation of the myocardium prior to application of the 1% ASB-14, Latrunculin, SARB sarcomere disassembly and solubilization protocol, results in a significant increase in the proportion of cardiomyocytes which are completely removed by the protocol, again ECM structure is retained, although some cardiomyocytes remain. **Seventh Row:** Relaxation of the myocardium followed by application of 2% ASB-14, Latrunculin and SARB results in complete removal of all cardiomyocytes and nuclei from the material. ECM matrix structure is completely retained.

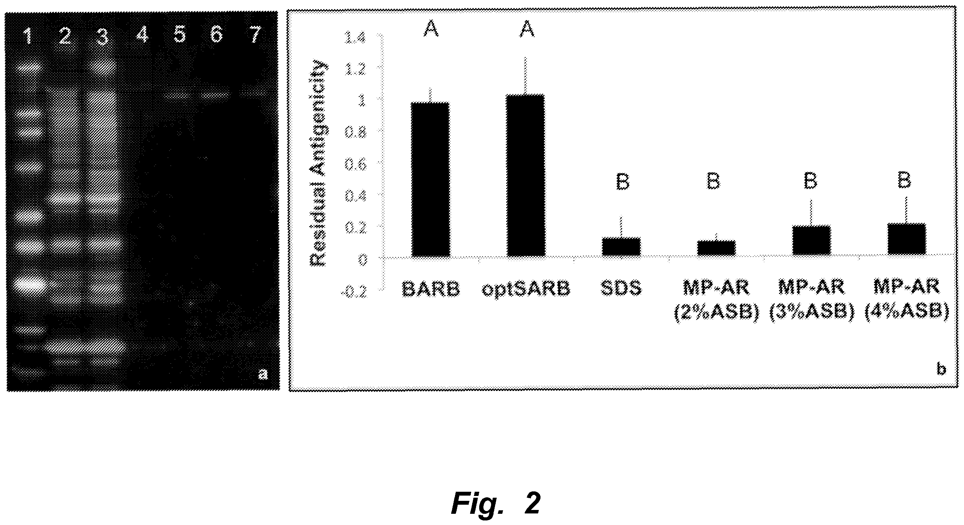

Figures 2A-B illustrate reduced levels of water-soluble antigens of left ventricular myocardial tissue using the methodologies described herein. Biomaterial residual antigenicity was assessed using Western blot methods. a; Western blot of residual antigens extracted from myocardial patches (MP) probed using mouse anti-rat LV poly-polyclonal serum. Lanes: 1; MagicMark Protein Ladder (Invitrogen), 2; BARB native tissue control, 3; optSARB, 4; 1%SDS, 5; myocardial patch following antigen removal (MP-AR) as described in the current invention (Step 1 for Water-Soluble antigens: LatrunculinB, KCl, KI; followed by Step 2 for Lipid-Soluble antigens: 2% ASB in optSARB), 6; MP-AR using 3% ASB in optSARB in the second step of AR, 7; MP-AR using 4% ASB in optSARB, b; Densitometry analysis for water-soluble residual antigenicity. Results displayed as means ± standard deviation. Our antigen removal approach (MP-AR) results in removal of >90% of antigens from the myocardial patch. Groups not connected by the same letter are significantly different, p < 0.01 (n=6 per group). 90% of water-soluble antigens were removed following MP-AR at various levels of ASB concentration (2% vs. 3% vs. 4%).

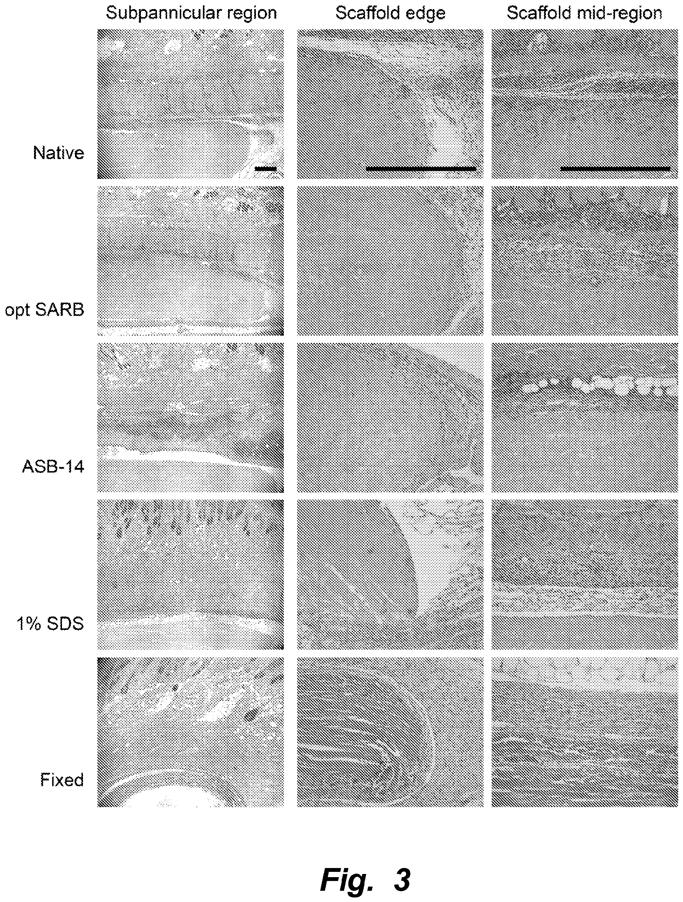

Figure 3 illustrates histological analysis of BP explants following one week of implantation in New Zealand White rabbits. Scale bar = 500 µm.

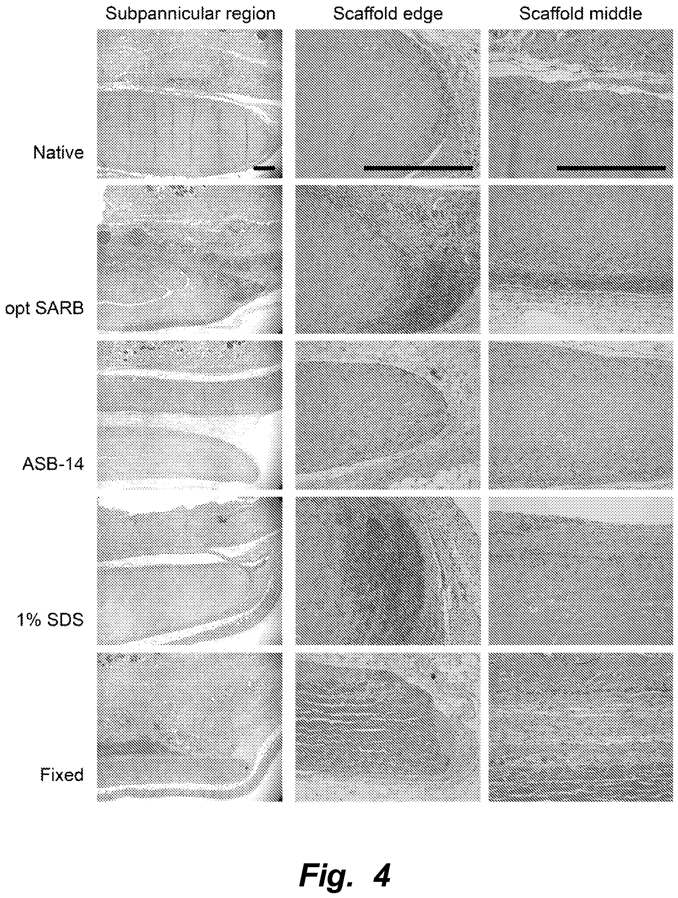

Figure 4 illustrates histological analysis of BP explants following six weeks of implantation in New Zealand White rabbits. Scale bar = 500 µm.

### DETAILED DESCRIPTION

### 1. Introduction

The present invention is based, in part, on methods for the sequential solubilization and extraction of cytoskeletal components (*e.g.,* filamentous actin and/or microtubules), water-soluble antigenic components and lipid-soluble antigenic components from tissue to produce tissue scaffold or decellularized extracellular matrix (ECM) with ECM structure, biochemical composition, mechanical properties and recellularization capacity that is substantially the same as the tissue prior to antigen removal procedures. Stepwise or sequential antigen removal of cytoskeletal components, water-soluble and lipid-soluble antigens from tissue substantially reduces *in vivo* immune response towards the produced tissue scaffold or decellularized ECM. Sequential solubilization and extraction of cytoskeletal components, water-soluble antigenic components and lipid-soluble antigenic components from tissue allows for the production of an immunologically-acceptable, structurally integral, mechanically sound, tissue scaffold compatible with recellularization, *e.g.,* for use in tissue engineering and/or tissue regeneration. When applied to muscle tissues, the methods can further comprise the step of relaxing, solubilizing and removing sarcomeric components.

Biomaterial antigenicity is the primary hurdle for the use of xenogeneic scaffolds in tissue engineering and regenerative medicine. To reduce or eliminate the persistent presence of antigens on decellularized tissue to elicit the immune response upon implantation we have developed a more rigorous antigen removal (AR) process. Solubilization-based AR has been shown to enhance the removal of water-soluble protein (WSP) antigens in tissues, including bovine pericardium (BP), beyond that achieved by decellularization using hypotonic solution or SDS (*e.g.,* at a concentration in the range of 0.1-1.0% w/v). However, the diversity of protein antigens within a tissue necessitates development of AR strategies capable of addressing a spectrum of protein antigen solubilities. The present invention is based, in part, on the discovery of antigen removal (AR) methods promoting the solubilization of lipid-soluble proteins (LSPs) to reduce the residual LSP antigenicity of tissues, including BP, when applied as a separate step of AR to solubilizing WSP. Promoting the solubilization of a protein subset (cytoskeletal components, WSPs and/or LSPs) significantly reduces the residual antigenicity of that specific subset of protein antigens (cytoskeletal components, WSPs and/or LSPs, respectively). However, promoting the solubilization of a protein subset (cytoskeletal components, WSPs and/or LSPs) does not significantly reduce residual antigenicity of the other subset of protein antigens (cytoskeletal components, WSPs and/or LSPs, respectively). Facilitating solubilization of cytoskeletal components (*e.g.,* by including one or more cytoskeletal destabilizing and/or depolymerizing agents), WSP (*e.g.,* using 100 mM dithiothreitol and 100 mM potassium chloride in solubilizing antigen removal buffer) and LSP (*e.g.,* using 1-4% (w/v) ASB-14 in solubilizing antigen removal buffer) in a multi-step sequential, differential AR strategy markedly reduces the residual antigenicity of tissues beyond that achieved with either one-step AR or decellularization by 1% (w/v) SDS. Use of 1-4% (w/v) ASB-14 for LSP AR does not compromise the biomaterial properties of tissue following antigen removal. A multi-step AR strategy promoting sequential, differential protein solubilization significantly reduced residual LSP antigens beyond that achieved with one-step AR of WSPs while maintaining biomaterial functional properties. Moreover, total DNA content and/or residual nuclei counts may not be an appropriate indicator of residual LSP antigenicity. This study demonstrates the importance of a sequential, differential protein solubilization approach for the reduction of biomaterial antigenicity in xenogeneic scaffold generation for tissue engineering.

For the application to muscle tissue, principles of protein chemistry can be applied to antigen removal of each antigenic class from muscle tissue. The methods entail a multimodal approach to disassemble and solubilize the myocyte sarcomere structure (the functional rigid force-producing units that make up myocytes). This approach produces more complete removal of antigens from myocytes than previously reported methods, while maintaining ECM structure-function relationships and improving recellularization capacity. The methods described herein produce decellularized tissue and ECM scaffolds with significantly reduced antigenicity, due to more complete removal of cytoskeletal, sarcomeric, hydrophilic and lipophilic antigens. This present methods employ macromolecular disassembly of the basic structural unit of myocytes (sarcomeres) combined with the principles of sequential, differential solubilization of water-soluble antigens and lipid-soluble antigens for sequential myocyte solubilization and antigen removal from muscle tissue (*e.g.,* cardiac muscle tissue, striated or skeletal muscle tissue, smooth muscle tissue).

The methods generally involve the treatment of allogeneic (*e.g.,* from a different individual of the same species of the subject receiving the tissue) and xenogeneic (*e.g.,* from a different species of the subject receiving the tissue) muscle tissues (*e.g.,* myocardial, skeletal or smooth muscle) for the purpose of removing cells, cellular debris, antigens, proteins, nucleic acids, phospholipids, and other macromolecules prior to implantation. The approach to production of such ECM scaffolds described herein is based on the protein chemistry principles of sequential, differential solubilization, utilized for protein extraction from homogenized tissues, *e.g.,* for proteomic applications. Protein solubilization is known to be important for achieving protein extraction from homogenized sample material for use in subsequent two-dimensional gel electrophoresis (2-DE). Protein precipitation tends to occur due to molecular interactions which result in protein aggregation (e.g., disulfide bond formation, hydrophobic interactions, non-covalent interactions). Consequently, disruption of macromolecular interactions within and between proteins is important for achieving and maintaining protein solubility. Therefore, the goal of most protein extraction protocols is to disaggregate, denature, reduce and consequentially solubilize the protein type of interest. Furthermore, various protein types within a tissue exhibit different physicochemical properties (*e.g.,* cytoplasmic proteins are generally water soluble, integral membrane proteins are generally hydrophobic). No single extraction condition is capable of simultaneously solubilizing all proteins within a tissue.

Protein extraction protocols, therefore, generally utilize sequential extraction techniques to solubilize proteins in a stepwise manner from the tissue by serial application of extraction solutions with characteristics designed to favor solubilization of a particular protein type. Previously reported decellularization and AR methods have largely failed to apply the principles of protein chemistry required to achieve solubilization of various antigenic protein types within the tissue. The present methods are based, in part, on the recognition that the macromolecular structure of the sarcomere prevents solubilization and subsequent removal of these components from the material. We therefore designed a specifically targeted stepwise approach to relax, depolymerize and render the components of the sarcomere amenable to solubilization. Combining sarcomere disassembly and solubilization with sequential, differential solubilization of water soluble antigens and lipid soluble antigens for antigen removal results in enhanced removal of cellular components and antigens from muscle tissues.

### 2. Methods For Removing Antigen Components From Tissue

The present methods relate to the removal of antigens from tissues, *e.g.,* cells, cellular debris, proteins, nucleic acids, phospholipids, carbohydrates and other macromolecules, *e.g.,* from tissues allogeneic (i.e., derived from within the same species) to or xenogeneic (i.e., derived from different species) to a recipient of the tissue, *e.g.,* in tissue or organ transplantation or regeneration.

### a. Tissues

The tissues subject to sequential antigen removal can be from any tissue suitable for transplantation. Generally, the tissue is live and unfixed. In various embodiments, tissues subject to sequential antigen removal include without limitation connective tissue, muscle tissue, nervous tissue (of the brain, spinal cord, and nerves), epithelial tissue, and organ tissue. Connective tissue includes fibrous tissue like fascia, tendon, ligaments, heart valves, bone, and cartilage. Muscle tissue includes skeletal muscle tissue, smooth muscle tissue, such as esophageal, stomach, intestinal, bronchial, uterine, urethral, bladder, and blood vessel tissue, and cardiac muscle tissue. Epithelial tissue includes simple epithelial tissue, such as alveolar epithelial tissue, blood vessel endothelial tissue, and heart mesothelial tissue, and stratified epithelial tissue. In various embodiments, the tissue is subject to antigen removal is heart, heart valve, vessel, vascular conduit, artery, vein, skin, dermis, pericardium, dura, intestinal submucosa, ligament, tendon, bone, cartilage, ureter, urinary bladder, liver, lung, and umbilical cord. The tissue may be a part of an organ or be an intact organ.

The tissue may be from the intended recipient (*e.g*., is syngeneic), from the same species as the intended recipient (*e.g*., is allogeneic) or from a different species from the intended recipient (*e.g*., is xenogeneic) of the tissue scaffold or decellularized ECM produced by removal of the antigens. In various embodiments, the tissue is from a first human and intended to be transplanted into second human. In various embodiments, the tissue is from a porcine, ovine, bovine, ostrich (*e.g.,* of the genus Struthio) or a non-human primate and intended to be transplanted into a human.

As appropriate, the tissue can be submerged in solubilization solution or perfused with solubilizing solution. Antigens can be effectively removed from thinner tissues (*e.g.,* tissues having about 1 mm thickness) by submerging. Antigens can be effectively removed from thicker tissues and intact organs by perfusion of the tissue with solubilizing solution.

Generally, the tissue is subjected to solubilization as soon as practicable after extraction from the original host and before the tissue is substantially decomposed. In various embodiments, the tissue is subjected to solubilization within 12 hours after extraction, *e.g.,* within 10, 8, 6, 4, 3, 2, 1 hours after extraction from the original host.

### b. Destabilizing and/or Depolymerizing Cytoskeletal Components

The tissue can be contacted with (*e.g.*, submerged in or perfused with) a solution for destabilizing and/or depolymerizing one or more cytoskeletal components (*e.g.*, filamentous actin and/or microtubules) under sufficient conditions and for a sufficient time to depolymerize, solubilize and extract a portion of the cytoskeletal components, *e.g.,* polymerized and/or filamentous cytoskeletal components. In various embodiments, the tissue is contacted with one or more agents that destabilize and/or depolymerize filamentous actin and/or microtubules.

In some embodiments, the one or more cytoskeletal destabilizing and/or depolymerizing agents comprise one or more actin destabilizing and/or depolymerization agents. In some embodiments, the one or more actin destabilizing and/or depolymerizing agents are selected from the group consisting of Cytochalasin A, Cytochalasin B, Cytochalasin C, Cytochalasin D, Cytochalasin E, Cytochalasin F, Cytochalasin G, Cytochalasin H, Cytochalasin I, Cytochalasin J, Latrunculin A, Latrunculin B, Swinholide A, Misakinolide A, Bistheonelide A, Scytophycin A, Scytophycin B, Scytophycin D, Scytophycin E, 19-0-Demethylscytophycin C, 6-Hydroxyscytophycin B, 6-Hydroxy-7-o-methylscytophycin E and tolytoxin, Mycalolide A, Mycalolide B, Mycalolide C, secomycalolide A and 30-hydroxymycalolide A, Halichondramide, (19Z)-halichondramide, kabiramides B, kabiramides C, kabiramides D, kabiramides G, kabiramides J, kabiramides K, ulapualide A, jaspamide, Dihydrohalichondramide, Aplyronine A, Aplyronine B, Aplyronine C, Pectenotoxin 2, Pectenotoxin 6, and Migrastatin. In some embodiments, the actin depolymerization agents depolymerize filamentous cytoskeletal actin (F-actin). In some embodiments, the actin depolymerization agents depolymerize filamentous α-sarcomeric actin (F-actin).

In some embodiments, the one or more cytoskeletal destabilizing and/or depolymerizing agents comprise one or more microtubule depolymerization and/or destabilizing agents. In some embodiments, the one or more microtubule depolymerization and/or destabilizing agents is selected from the group consisting of colchicine, colcemid, vinblastine, vincristine, myoseverin, nocodazole, podophyllotoxin, polygamain and taxol.

In various embodiments, the solution comprising the one or more cytoskeletal component destabilizing and/or depolymerizing agents is an aqueous solution. In varying embodiments, the solution comprising the one or more cytoskeletal component destabilizing and/or depolymerizing agents is a physiologically isotonic aqueous solution (e.g., serum free cell culture solution). In varying embodiments, the one or more cytoskeletal component destabilizing and/or depolymerizing agents is added to a solution for solubilizing the water-soluble antigens, as described below, in the section entitled "Solubilizing Water-Soluble Antigens in the Tissue." In some embodiments, the aqueous solution comprises glucose, *e.g.,* at a concentration in the range of about 5-50 mM, *e.g.,* about 5-25 mM, *e.g.,* about 5 mM, 10 mM, 15 mM, 20 mM, 25 mM, 30 mM, 35 mM, 40 mM, 45 mM, or 50 mM.

In varying embodiments, the tissue is contacted with (*e.g.,* is submerged in or perfused with) each cytoskeletal destabilizing and/or depolymerizing agent at a concentration in the range of about 1 nM to about 10 µM, *e.g.,* at a concentration of about 1 nM, 5 nM, 10 nM, 25 nM, 50 nM, 75 nM, 100 nM, 500 nM, 750 nM, 1 µM, 5 µM, 10 µM, 15 µM, 20 µM, 25 µM, 30 µM, 35 µM, 40 µM, 45 µM and 50 µM.

In various embodiments, the tissue is submerged in or perfused with the solution comprising one or more cytoskeletal depolymerizing and/or destabilizing agents for at least about 0.5, 1, 2, 4, 6, 8, 10, 12, 14, 16, 18, 20, 22 or 24 hours, *e.g.,* for at least about 2, 3, 4, 5, 6, 7 days, as appropriate. The tissue may be washed one or more times during the time period of submerging or perfusing, *e.g.,* to promote diffusion and separation of cytoskeletal antigens from the tissue.

In various embodiments, the depolymerization, solubilization, extraction and/or removal of depolymerized cytoskeletal components is performed at a temperature above freezing (*e.g.,* above 0°C) and at or below body temperature (*e.g.,* at or below about 37°C). In various embodiments, the depolymerization, solubilization, extraction and/or removal of depolymerized cytoskeletal components is performed at a refrigerated temperature, *e.g.,* between about 4-10°C. In various embodiments, the depolymerization, solubilization, extraction and/or removal of depolymerized cytoskeletal components is performed at room temperature, *e.g.,* between about 20-30°C, *e.g.,* about 25°C. In various embodiments, the depolymerization, solubilization, extraction and/or removal of depolymerized cytoskeletal components is performed at human body temperature, e.g., about 37°C. The depolymerization, solubilization, extraction and/or removal steps can be performed at the same or different temperatures.

In performing the methods, the step of contacting the tissue with a solution comprising one or more cytoskeletal destabilizing and/or depolymerizing agents can be conducted concurrently with or in a separate step from the step of contacting the tissues with a solution for solubilizing the water-soluble antigens. After a portion of the cytoskeletal components are depolymerized, solubilized and extracted from the tissue, the solubilized and extracted cytoskeletal components are separated from the tissue, *e.g.,* in a wash step. In various embodiments, washing is performed by contacting tissue *(*e.g., submerging in or perfusing with) with fresh physiologically isotonic solution *(*e.g., serum free cell culture solution) or a solution for solubilizing the water-soluble antigens, as described below. The step of depolymerizing, solubilizing and extracting cytoskeletal components can be performed for one or multiple iterations, *e.g.,* 2, 3, 4, 5, or more iterations, as appropriate. For example, the iterations of solubilizing depolymerized cytoskeletal components and separating *(e.g.,* rinsing) the cytoskeletal components from the tissue can be repeated until extracted cytoskeletal components are no longer detected in the depolymerizing solution or the tissue, or until the detectable cytoskeletal components in the extraction solution or the tissue fall below a predetermined threshold level, as appropriate.

### c. Solubilizing Water-Soluble Antigens In The Tissue

The tissue can be contacted with (*e.g*., submerged in or perfused with) a solution for solubilizing the water-soluble antigens under sufficient conditions and for a sufficient time to extract a portion of the water soluble antigens from the tissue or to reach an equilibrium between the water-soluble antigens within the tissue and water soluble antigens in the solution, as appropriate.

In various embodiments, the tissue is submerged in or perfused with the solution for solubilizing the water-soluble antigens for at least about 0.5, 1, 2, 4, 6, 8, 10, 12, 14, 16, 18, 20, 22 or 24 hours, *e.g.,* for at least about 2, 3, 4, 5, 6, 7 days, as appropriate. The tissue may be washed one or more times during the time period of submerging or perfusing, *e.g.,* to promote diffusion and separation of water-soluble antigens from the tissue.

In various embodiments, removal of water-soluble antigens is performed at a temperature above freezing (*e.g.,* above 0°C) and at or below body temperature (*e.g.,* at or below about 37°C). In various embodiments, removal of water-soluble antigens is performed at a refrigerated temperature, *e.g.,* between about 4-10°C. In various embodiments, removal of water-soluble antigens is performed at room temperature, *e.g.,* between about 20-30°C, *e.g.,* about 25°C. In various embodiments, removal of water-soluble antigens is performed at human body temperature, *e.g.,* about 37°C.

The water-soluble antigens are solubilized in a solution comprising a buffering agent, a reducing agent, a protease inhibitor, and one or more salts suitable for maintaining protein solubility.

In various embodiments, the buffering agent maintains a pH (*e.g.,* has a pKa) to allow for solubility of the antigens in aqueous solution. For example, in some embodiments, the buffering agent maintains a pH in the range of about 4-11, e.g., a pH in the range of about 4-6, 8-11, 5-10 or 6-9. In some embodiments, the buffering agent maintains a pH of at least about 8. In some embodiments, the buffering agent maintains a pH of less than about 5. Illustrative buffering agents include without limitation Tris-HCl, phosphate, citric acid, acetate, imidazole, carbonate, MES, Bis-Tris, ADA, aces, PIPES, MOPSO, Bis-Tris propane, BES, MOPS, TES, HEPES, DIPSO, MOBS, TAPSO, Trizma, HEPPSO, POPSO, TEA, EPPS, Tricine, Gly-Gly, Bicine, HepBS, TAPS, AMPD, CHES, CAPSO, AMP, CAPS and CABS. These and other buffering agents of use are well-known in the art and commercially available, *e.g.,* from Sigma-Aldrich (on the internet at sigmaaldrich.com). In some embodiments, the buffering is Tris-HCl.

In some embodiments, the one or more salts comprise a monovalent or a divalent anion. In some embodiments, the one or more salts comprise a metal halide salt. Illustrative metal halide salts of use include without limitation LiF, LiCl, LiBr, LiI, NaF, NaCl, NaBr, NaI, KF, KCl, KBr, KI, RbF, RbCl, RbBr, RbI, CsF, CsCl, CsBr, CsI, BeF₂, BeCl₂, BeBr₂, BeI₂, MgF₂, MgCl₂, MgBr₂, MgI₂, CaF₂, CaCl₂, CaBr₂, CaI₂, SrF₂, SrCl₂, SrBr₂, SrI₂, BaF₂, BaCl₂, BaBr₂, BaI₂, and mixtures thereof. In some embodiments, the one or more salts comprise KCl. In various embodiments, the one or more salts are included at a concentration of at least about 50 mM, 75 mM, or 100 mM, for example, at least about 150 mM, 200 mM, 250 mM, 300 mM, 350mM, 400mM, 450mM, 500 mM. 600 mM, 700 mM, 800 mM, 900 mM or 1000 mM, for example, in the range of about 100-500 mM or about 100-200 mM.

Illustrative reducing agents for use in the solution for solubilizing the water-soluble antigens include without limitation Tributylphosphine (TBP), beta mercaptoethanol, 2-mercaptoethylamine, *tris*(2-carboxyethyl)phosphine (TCEP), cysteine-HCl, and dithiothreitol (DTT). In some embodiments, the reducing agent is DTT.

Illustrative protease inhibitors for use in the solution for solubilizing the water-soluble antigens include without limitation aspartic protease inhibitors, cysteine protease inhibitors, metalloprotease inhibitors, serine protease inhibitors (serpins), threonine protease inhibitors, trypsin inhibitors, and mixtures thereof. In various embodiments, the protease inhibitor is an I9, I10, I14, I24, I29, I34, I36, I42, I48, I53, I67, I68, 178 inhibitor, or a mixture thereof. In some embodiments, the protease inhibitor is AEBSF (4-(2-Aminoethyl) benzenesulfonyl fluoride hydrochloride), also sold as PEFABLOC®. In some embodiments, the protease inhibitor is Phenylmethylsulfonyl fluoride (PMSF). Numerous protease inhibitor cocktails of use are commercially available from Roche Molecular Biochemicals.

In some embodiments, the water-soluble antigens are solubilized in a solution that comprises one or more of an antibacterial agent and/or an antifungal agent.

In some embodiments, the water-soluble antigens are solubilized in a solution that comprises a chelation agent. Illustrative chelation agents include without limitation ethylenediaminetetraacetic acid (EDTA), diethylenetriaminepentaacetic acid (DTPA), Citric Acid, N,N-bis(carboxymethyl)glycine (NTA), and the meso isomer of Dimercaptosuccinic acid (DMSA).

In some embodiments, the water-soluble antigens are solubilized in a solution that does not comprise an amphiphile. In some embodiments, the water-soluble antigens are solubilized in a solution that does not comprise a detergent. In some embodiments, the water-soluble antigens are solubilized in a solution that comprises a non-detergent sulfobetaine. Illustrative non-detergent sulfobetaines include without limitation NDSB-256, NDSB-211, NDSB-195, NDSB-221 and NDSB-201.

In some embodiments, the water-soluble antigens are solubilized in a solution comprising Tris-HCl, dithiothreitol (DTT), a protease inhibitor, and KCl. In a particular embodiment, the water-soluble antigens are solubilized in a solution comprising 10 mM Tris-HCl, 100 mM DTT, 100 mM KCl and 2mM MgCl₂. A protease inhibitor and/or an antibacterial agent and/or an antifungal agent may also be included.

### d. Separating The Tissue From The Solubilized Water-Soluble Antigens

Once the tissue has been submerged in or perfused with the solution for solubilizing the water-soluble antigens under sufficient conditions and for a sufficient time, the tissue can be separated from the solution, now containing a portion of the water soluble antigens extracted from the tissue. Separation of the tissue and the solubilizing solution can be performed using any methods known in the art. In various embodiments, the tissue can be rinsed, *e.g.,* in the same solution for solubilizing the water-soluble antigens. Optionally, the tissue can be subject to saturation with (*e.g.,* submerged in or perfused with) solubilization solution and separated for one or more iterations in fresh solution for solubilizing the water-soluble antigens for a sufficient time to extract a further portion of the water soluble antigens from the tissue or to reach an equilibrium between the water-soluble antigens within the tissue and water soluble antigens in the solution, as appropriate. The iterations of submerging in or perfusing with solution for solubilizing the water-soluble antigens and separating (*e.g.,* rinsing) can be repeated until extracted water-soluble antigens are no longer detected in the solution for solubilizing the water-soluble antigens, or until the detectable extracted water-soluble antigens fall below a predetermined threshold level, as appropriate.

In various embodiments, the separation and removal of the water-soluble antigens from the tissue can involve centrifugation and/or filtration, as appropriate, to separate the tissue from the solution for solubilizing the water-soluble antigens containing a portion of and/or saturated with extracted water-soluble antigens. In other embodiments, the tissue is removed from solution for solubilizing the water-soluble antigens and rinsed with being subjected to centrifugation or filtration.

In various embodiments, more frequent iterations of submerging or perfusing and separation (*e.g.,* rinsing) and a larger number of iterations of submerging or perfusing and separating (*e.g.,* rinsing) can allow for faster solubilization and removal of water-soluble antigens from the tissue. For example, the tissue can be saturated with (*e.g.,* submerged in or perfused with) a first solution for solubilizing the water-soluble antigens for a sufficient time to extract at least a portion of the water soluble antigens; separated from the first solution, now containing a portion of extracted water soluble antigens, before equilibrium is reached between the water-soluble antigens within the tissue and water soluble antigens in the solution; saturated with (*e.g.,* submerged in or perfused with) a second solution for solubilizing the water-soluble antigens for a sufficient time to extract at least a portion of the water soluble antigen; and separated from the second solution, now containing a portion of extracted water soluble antigens, before equilibrium is reached between the water-soluble antigens within the tissue and water soluble antigens in the solution. Further iterations of submerging or perfusing and separation can be performed, as appropriate or desired, until a sufficiently low level of water soluble antigens extracted into the solution for solubilizing the water-soluble antigens is achieved.

### e. Solubilizing Lipid-Soluble Antigens In The Tissue

In various embodiments, tissues that have been subject to extraction and separation of water-soluble antigens can subsequently be subject to extraction of lipid-soluble antigens. In other embodiments, tissues are first subject to extraction of lipid soluble antigens and second subject to extraction of water-soluble antigens.

The tissue can be saturated with (*e.g.,* submerged in or perfused with) a solution for solubilizing the lipid-soluble antigens for a sufficient time to extract a portion of the lipid soluble antigens from the tissue or to reach an equilibrium between the lipid-soluble antigens within the tissue and lipid soluble antigens in the solution, as appropriate.

In various embodiments, the tissue is saturated with (*e.g.,* submerged in or perfused with) the solution for solubilizing the lipid-soluble antigens for at least about 0.5, 1, 2, 4, 6, 8, 10, 12, 14, 16, 18, 20, 22 or 24 hours, *e.g.,* for at least about 2, 3, 4, 5, 6, 7 days, as appropriate. The tissue may be washed one or more times during the time period of submerging or perfusing, *e.g.,* to promote diffusion and separation of lipid-soluble antigens from the tissue.

In various embodiments, removal of lipid-soluble antigens is performed at a temperature above freezing *(e.g.,* above 0°C) and at or below body temperature (*e.g.,* at or below about 37°C). In various embodiments, removal of lipid-soluble antigens is performed at a refrigerated temperature, *e.g.,* between about 4-10°C. In various embodiments, removal of lipid-soluble antigens is performed at room temperature, *e.g.,* between about 20-30°C, *e.g.,* about 25°C. In various embodiments, removal of lipid-soluble antigens is performed at human body temperature, *e.g.,* about 37°C.

In some embodiments, the lipid-soluble antigens are solubilized in a solution comprising a buffering agent, a reducing agent, a protease inhibitor, one or more salts suitable for maintaining protein solubility and an amphiphile.

In various embodiments, the amphiphile is a zwitterionic detergent. In some embodiments, the amphiphile is a sulfobetaine. Illustrative sulfobetaines of use include without limitation 3-[N,N-Dimethyl(3-myristoylaminopropyl)ammonio]propanesulfonate (amidosulfobetaine-14; ASB-14); amidosulfobetaine-16 (ASB-16); 4-n-Octylbenzoylamido-propyl-dimethylammonio sulfobetaine (ASB-C8Ø); 3-(N,N-Dimethyloctylammonio) propanesulfonate inner salt (SB3-8); N-Decyl-N,N-dimethyl-3-ammonio-1-propanesulfonate (SB 3-10), N-Decyl-N,N-dimethyl-3-ammonio-1-propanesulfonate (SB 3-12), N-Tetradecyl-N,N-dimethyl-3-ammonio-1-propanesulfonate (SB 3-14); 3-(N,N-Dimethylpalmitylammonio) propanesulfonate (SB3-16); 3-(N,N-Dimethyloctadecylammonio) propanesulfonate (SB3-18); 3-(1-Pyridinio)-1-propanesulfonate (NDSB-201); 3-(Benzyldimethylammonio) propanesulfonate (NDSB-256); NDSB-211, NDSB-195, NDSB-221; 3-[(3-Cholamidopropyl)dimethylammonio]-1-propanesulfonate (CHAPS), 3-[(3-Cholamidopropyl)dimethylammonio]-2-hydroxy-1-propanesulfonate (CHAPSO), and mixtures thereof. In various embodiments, the sulfobetaine is an amidosulfobetaine. Illustrative amidosulfobetaines include without limitation, ASB-14, ASB-16 and ASB- C8Ø. In various embodiments, the sulfobetaine is a non-detergent sulfobetaine. Illustrative non-detergent sulfobetaines include without limitation NDSB-256, NDSB-211, NDSB-195, NDSB-221 and NDSB-201.

In various embodiments, the buffering agent maintains a pH (*e.g.,* has a pKa) to allow for solubility of the antigens in aqueous solution. For example, in some embodiments, the buffering agent maintains a pH in the range of about 4-11, *e.g.,* a pH in the range of about 4-6, 8-11, 5-10 or 6-9. In some embodiments, the buffering agent maintains a pH of at least about 8. Illustrative buffering agents include without limitation Tris-HCl, phosphate, citric acid, acetate, imidazole, carbonate, MES, Bis-Tris, ADA, aces, PIPES, MOPSO, Bis-Tris propane, BES, MOPS, TES, HEPES, DIPSO, MOBS, TAPSO, Trizma, HEPPSO, POPSO, TEA, EPPS, Tricine, Gly-Gly, Bicine, HepBS, TAPS, AMPD, CHES, CAPSO, AMP, CAPS and CABS. These and other buffering agents of use are well-known in the art and commercially available, *e.g.,* from Sigma-Aldrich (on the internet at sigmaaldrich.com). In some embodiments, the buffering is Tris-HCl.

In some embodiments, the one or more salts comprise a monovalent or a divalent anion. In some embodiments, the one or more salts comprise a metal halide salt. Illustrative metal halide salts of use include without limitation LiF, LiCl, LiBr, LiI, NaF, NaCl, NaBr, NaI, KF, KCl, KBr, KI, RbF, RbCl, RbBr, RbI, CsF, CsCl, CsBr, CsI, BeF₂, BeCl₂, BeBr₂, BeI₂, MgF₂, MgCl₂, MgBr₂, MgI₂, CaF₂, CaCl₂, CaBr₂, CaI₂, SrF₂, SrCl₂, SrBr₂, SrI₂, BaF₂, BaCl₂, BaBr₂, BaI₂, and mixtures thereof. In some embodiments, the one or more salts comprise KCl. In various embodiments, the one or more salts are included at a concentration of at least about 50mM, 75mM, or 100 mM, for example, at least about 150 mM, 200 mM, 250 mM, 300 mM, 350mM, 400mM, 450mM or 500 mM.

Illustrative reducing agents for use in the solution for solubilizing the lipid-soluble antigens include without limitation Tributylphosphine (TBP), beta mercaptoethanol, 2-mercaptoethylamine, *tris*(2-carboxyethyl)phosphine (TCEP), cysteine-HCl, and dithiothreitol (DTT). In some embodiments, the reducing agent is DTT.

Illustrative protease inhibitors for use in the solution for solubilizing the lipid-soluble antigens include without limitation aspartic protease inhibitors, cysteine protease inhibitors, metalloprotease inhibitors, serine protease inhibitors (serpins), threonine protease inhibitors, trypsin inhibitors, and mixtures thereof. In various embodiments, the protease inhibitor is an I9, I10, I14, I24, I29, I34, I36, I42, I48, I53, I67, I68, I78 inhibitor, or a mixture thereof. In some embodiments, the protease inhibitor is AEBSF (4-(2-Aminoethyl) benzenesulfonyl fluoride hydrochloride), also sold as PEFABLOC®. In some embodiments, the protease inhibitor is Phenylmethylsulfonyl fluoride (PMSF). Numerous protease inhibitor cocktails of use are commercially available from Roche Molecular Biochemicals.

In some embodiments, the lipid-soluble antigens are solubilized in a solution that comprises one or more of an antibacterial agent and/or an antifungal agent.

In some embodiments, the lipid-soluble antigens are solubilized in a solution that comprises a chelation agent. Illustrative chelation agents include without limitation ethylenediaminetetraacetic acid (EDTA), diethylenetriaminepentaacetic acid (DTPA), Citric Acid, N,N-bis(carboxymethyl)glycine (NTA), and the meso isomer of Dimercaptosuccinic acid (DMSA).

In some embodiments, the lipid-soluble antigens are solubilized in a solution comprising Tris-HCl, dithiothreitol (DTT), a protease inhibitor, KCl and ASB-14. In a particular embodiment, the lipid-soluble antigens are solubilized in a solution comprising 10 mM Tris-HCl, 100 mM DTT, 100 mM KCl, 2mM MgCl₂ and 1-4% ASB-14. A protease inhibitor and/or an antibacterial agent and/or an antifungal agent may also be included.

### f. Separating The Tissue From The Solubilized Lipid-Soluble Antigens

Once the tissue has been saturated with (*e.g.,* submerged in or perfused with) the solution for solubilizing the lipid-soluble antigens for a sufficient time, the tissue can be separated from the solution, now containing a portion of the lipid soluble antigens extracted from the tissue. Separation of the tissue and the solubilizing solution can be performed using any methods known in the art. In various embodiments, the tissue can be rinsed, *e.g.,* in the same solution for solubilizing the lipid-soluble antigens. Optionally, the tissue can be saturated with (*e.g.,* submerged in or perfused with) and separated for one or more iterations in fresh solution for solubilizing the lipid-soluble antigens for a sufficient time to extract a further portion of the lipid soluble antigens from the tissue or to reach an equilibrium between the lipid-soluble antigens within the tissue and lipid soluble antigens in the solution, as appropriate. The iterations of submerging or perfusing in solution for solubilizing the lipid-soluble antigens and separating (*e.g.,* rinsing) can be repeated until extracted lipid-soluble antigens are no longer detected in the solution for solubilizing the lipid-soluble antigens, or until the detectable extracted lipid-soluble antigens fall below a predetermined threshold level, as appropriate.

In various embodiments, the separation and removal of the lipid-soluble antigens from the tissue can involve centrifugation and/or filtration, as appropriate, to separate the tissue from the solution for solubilizing the lipid-soluble antigens containing a portion of and/or saturated with extracted lipid-soluble antigens. In other embodiments, the tissue is removed from solution for solubilizing the lipid-soluble antigens and rinsed with being subjected to centrifugation or filtration.

In various embodiments, more frequent iterations of submerging or perfusing and separation (*e.g.,* rinsing) and a larger number of iterations of submerging or perfusing and separating (*e.g.,* rinsing) can allow for faster solubilization and removal of lipid-soluble antigens from the tissue. For example, the tissue can be saturated with (*e.g.,* submerged in or perfused with) a first solution for solubilizing the lipid-soluble antigens for a sufficient time to extract at least a portion of the lipid soluble antigens; separated from the first solution, now containing a portion of extracted lipid soluble antigens, before equilibrium is reached between the lipid-soluble antigens within the tissue and lipid soluble antigens in the solution; saturated with (*e.g.,* submerged in or perfused with) a second solution for solubilizing the lipid-soluble antigens for a sufficient time to extract at least a portion of the lipid soluble antigen; and separated from the second solution, now containing a portion of extracted lipid soluble antigens, before equilibrium is reached between the lipid-soluble antigens within the tissue and lipid soluble antigens in the solution. Further iterations of submerging or perfusing and separation can be performed, as appropriate or desired, until a sufficiently low level of lipid soluble antigens extracted into the solution for solubilizing the lipid-soluble antigens is achieved.

### g. Recellularizaton/Repopulation with Live Cells

In various embodiments, the tissue scaffold and/or decellularized ECM (*e.g.,* heart valves, vascular conduits, arteries, veins, skin, dermis, ligaments, tendons, bone, cartilage, muscle, ureter, urinary bladder, liver, heart, and other organs) can be recellularized or repopulated prior to implantation by co-culturing the tissue processed according to a method of the invention with live cells, *e.g.,* cells autologous to the recipient of the tissue; cells of the same tissue type as the tissue to be transplanted into the recipient; mesenchymal stem cells; and mixtures thereof.

In various embodiments, the tissue scaffold and/or decellularized ECM is repopulated or recellularized with mesenchymal stem cells exhibit immunomodulatory properties through mechanisms involving both cell-to-cell contact and secretion of soluble factors (PGE2, TGF-β1, IL-6 and hepatocyte growth factor (HGF)). Specifically, in the allogeneic setting, MSCs induce T-cell anergy, reduce dendritic cell type 1 (DC1) TNF-α secretion, increase DC2 IL-10 secretion, decrease T-helper cell type 1 (Th1) IFN-γ secretion, increase Th2 IL-4 secretion, increase the proportion of regulatory T-cells (Treg) and decrease natural killer (NK) cell IFN-γ secretion. Furthermore, previous studies have demonstrated the ability of MSCs to reconstitute all heart valve layers, making them a potentially ideal source for recellularization of a tissue engineered heart valve scaffold. Finally, MSCs exhibit multipotent capacity, autologous availability, clinical relevance and increased proliferative capacity compared to terminally differentiated cells. Recellularizaton with MSCs complements antigen removal in producing an immunologically-acceptable, recellularized tissue scaffold.

As appropriate, *in vitro* co-culture conditions can, in specific embodiments, be under static conditions for cell culture or can take place in a bio-reactor mimicking certain desired *in vivo* conditions. As mentioned above, processed tissue can, in some embodiments, be treated with growth factors (e.g., basic fibroblast growth factor) or chemokines to enhance cellular ingrowth/migration into the tissue and/or to direct cells to adopt appropriate phenotypes. Such treatments could be employed to enhance *in vitro* recellularization before implantation of the recellularized tissue scaffold or ECM or to enhance *in vivo* recellularization after implantation of the tissue scaffold and/or decellularized ECM, and would be familiar to the ordinarily skilled artisan.

In further embodiments, the tissue scaffolds and/or decellularized ECM can be treated or impregnated with agents, *e.g.,* growth factors and/or pharmaceuticals. Growth factors may, for example, be used to promote recellularization, vascularization, or epithelialization. Antibodies or antibiotics may be used to prevent potential infection from implant. Matrix components may also be used. Other so-called recellularization agents include, without limitation, chemoattractants, cytokines, chemokines, and derivatives thereof.

### 3. Decellularization and Antigen Removal from Muscle Tissue

In embodiments where the tissue is a muscle tissue, *e.g.,* cardiac muscle tissue, striated or skeletal muscle tissue, or smooth muscle tissue, the methods can further comprise the steps of relaxing the muscle tissue and washing the muscle tissue in a concentrated salt solution to disassemble and remove the sarcomeric components.

Muscle tissue, including cardiac muscle tissue, contains four main cell types: endothelial cells, fibroblasts, myocytes and smooth muscle cells. Cardiomyocytes have the highest percentage in volume of myocardium. Cardiomyocytes, as well as myocytes in striated or skeletal muscle tissue, are made up of sarcomeres aligned in series and in parallel. Since actin filaments represent an important component of the sarcomere structure, we investigated the addition of an actin depolymerization step (*e.g.,* utilizing swinholide, mycalolide B, Latrunculin B and/or Cytochalasin D) to convert filamentous cytoskeletal (cortical) and α-sarcomeric actin (F-actin) to globular actin monomers (G-actin). Conversion of F-actin to G-actin monomers increased solubility and therefore the removal of actin monomers, associated cytoskeletal proteins and antigens. Actin depolymerization alone was insufficient to facilitate complete solubilization of all macromolecular components of the sarcomere. An integrated multimodal approach is employed that complements the actin depolymerization step with additional steps specifically targeted to remove sarcomeric myosin and titin. Bathing muscle tissue in a basic "relaxing solution" designed to render the myosin heads on the sarcomeric apparatus in the relaxed state (*e.g.,* switched from the ADP-bound or "rigor state" to the ATP-bound or relaxed state) prior to the antigen removal improved removal of sarcomeric proteins and associated antigens from the resulting scaffold. In varying embodiments, antigen removal and decellularization of muscle tissue comprises the step of relaxation of the muscle tissue prior to the antigen removal process, followed by removal of lipid-soluble proteins/antigens, sarcomeric depolymerization and removal of specific sarcomeric constituents, and finally removal of water-soluble proteins/antigens. In varying embodiments, antigen removal and decellularization of muscle tissue comprises the step of relaxation of the muscle tissue prior to the antigen removal process, followed by sarcomeric depolymerization and removal of specific sarcomeric constituents, removal of water-soluble proteins/antigens and finally removal of lipid-soluble proteins/antigens. This multi-targeted approach results in successful removal of essentially all detectable macromolecular sarcomeric components and has a significant impact on removal of both sarcomere-associated, and non-sarcomere-associated antigens from the material.

### a. Relaxation of Muscle Tissue

The muscle tissue is contacted with (*e.g.,* submerged in or perfused with) a relaxing solution comprising an energy source molecule and/or a chemical phosphatase and/or myosin light chain kinase (MLCK) inhibitor under sufficient conditions and for a sufficient time to render the myocyte sarcomere structure (*e.g.,* the functional rigid force-producing units that make up myocytes) amenable to subsequent disassembly and solubilization. In the relaxation step, a substantial portion of the myosin heads on the sarcomeric apparatus are switched from the ADP-bound or "rigor state" to the ATP-bound or relaxed state. In varying embodiments, the energy source molecule is selected from the group consisting of a nucleotide 5'-triphosphate (NTP), adenosine, inosine, aspartate, glutamate, creatine phosphate, a Kreb's cycle precursor or intermediate, glucose, and dextrose. In some embodiments, the energy source molecule is a nucleotide 5'-triphosphate (NTP) selected from the group consisting of adenosine 5'-triphosphate (ATP), inosine 5'-triphosphate (ITP), guanidine 5'-triphosphate (GTP), cytidine 5'-triphosphate (CTP), and uridine 5'-triphosphate (UTP). In some embodiments, the energy source molecule is a precursor of adenosine 5'-triphosphate (ATP). In some embodiments, the energy source molecule is adenosine 5'-triphosphate (ATP). In varying embodiments, the chemical phosphatase and/or myosin ATPase inhibitor is selected from 2,3-Butanedione monoxime (BDM), blebbisatin, vanadate ions, sodium orthovanadate. In varying embodiments, the chemical phosphatase and/or myosin ATPase inhibitor is 2,3-Butanedione monoxime (BDM). In varying embodiments, the chemical phosphatase is selected from the group consisting of 2,3-Butanedione monoxime (BDM), NSC 87877, Microcystin-LA, Microcystin-LR, Microcystin-RR, β-Glycerophosphate (sodium salt hydrate), Cantharidin, bpV(phen) (potassium hydrate), bpV(pic) (potassium hydrate), bpV(HOpic) (potassium salt) and Okadaic Acid, purchasable, *e.g.,* from Cayman Chemical. In some embodiments, the myosin light chain kinase (MLCK) inhibitor is selected from N-(2-Aminoethyl)-5-chloronaphthalene-1-sulfonamide-HCl, ML-7, ML-9, K252a, K252b, Go7874-HCl, myosin light chain kinase inhibitor peptide 18, piceatannol, and/or staurosporine.

In varying embodiments, the relaxing solution comprises each of the one or more energy source molecules and/or a chemical phosphatases and/or myosin light chain kinase (MLCK) inhibitors in a concentration range of about 1 mM to about 200 mM, *e.g.,* about 1 mM, 2 mM, 3 mM, 4 mM, 5 mM, 6 mM, 7 mM, 8 mM, 9 mM, 10 mM, 25 mM, 50 mM, 75 mM, 100 mM, 125 mM, 150 mM, 175 mM, or 200 mM. In varying embodiments, the relaxing solution comprises 4 mM adenosine 5'-triphosphate (ATP). In varying embodiments, the relaxing solution comprises 50-200 mM glucose, glutamate and/or aspartate.

In varying embodiments, the relaxing solution is a physiologically isotonic aqueous solution. In some embodiments, the relaxing solution promotes disassembly of sarcomeric macromolecules as well as the solubilization of water soluble antigens, as described above in the section entitled "Solubilizing Water-Soluble Antigens in the Tissue," further comprising one or more energy source molecules.

In various embodiments, the tissue is submerged in or perfused with the relaxing solution for at least about 0.5, 1, 2, 4, 6, 8, 10, 12, 14, 16, 18, 20, 22 or 24 hours, *e.g.,* for at least about 2, 3, 4, 5, 6, 7 days, as appropriate. The tissue may be washed one or more times during the time period of submerging or perfusing, *e.g.,* to promote disassembly of sarcomeric components within the muscle tissue.

In various embodiments, relaxation of the muscle tissue is performed at a temperature above freezing (*e.g.,* above 0°C) and at or below body temperature (*e.g.,* at or below about 37°C). In various embodiments, relaxation of the muscle tissue is performed at a refrigerated temperature, *e.g.,* between about 4-10°C. In various embodiments, relaxation of the muscle tissue is performed at room temperature, *e.g.,* between about 20-30°C, *e.g.,* about 25°C. In various embodiments, relaxation of the muscle tissue is performed at human body temperature, *e.g.,* about 37°C.

In some embodiments, the relaxing solution further comprises a calcium ion chelating agent. Illustrative chelation agents include, without limitation, ethylenediaminetetraacetic acid (EDTA), diethylenetriaminepentaacetic acid (DTPA), Citric Acid, N,N-bis(carboxymethyl)glycine (NTA), and the meso isomer of Dimercaptosuccinic acid (DMSA). In some embodiments, the relaxing solution further comprises a permeabilization agent. In varying embodiments, the permeabilization is a detergent, *e.g,* as described above and herein. Illustrative detergents for use as permeabilization agents include without limitation, *e.g.,* Triton-X-100, saponin and/or a sulfobetaine (*e.g.,* ASB-14).

In a particular embodiment, the relaxing solution comprises one or more cytoskeletal destabilizing agents in a solution comprising 10 mM TrisHCl, pH 7.6; 0.12 M KCl; 4 mM MgCl₂ 6 H₂O; 4 mM EDTA (hydrated) and 4-6 mM energy source molecule (*e.g.,* Na-ATP or ATP)). In varying embodiments, the relaxing solution may further comprise a protease inhibitor and one or more antimicrobial agents, *e.g.,* 0.5 mM AEBSF (4-(2-Aminoethyl) benzenesulfonyl fluoride hydrochloride) ("PEFABLOC™") and 1% Antibiotic Antimycotic Solution (AAS).

### b. High Salt Concentration Wash to Facilitate Removal of Sarcomeric Components

In varying embodiments, the muscle tissue is exposed to (*e.g.,* submerged in or perfused with) a concentrated salt solution to facilitate the solubilization and removal of sarcomeric components. In varying embodiments, the concentrated salt solution comprises one or more salts in a concentration range from about 0.5 M to about 3.0 M, *e.g.,* about 0.6 M, 0.7 M, 0.8 M, 0.9 M, 1.0 M, 1.1 M, 1.2 M, 1.3 M, 1.4 M, 1.5 M, 1.6 M, 1.7 M, 1.8 M, 1.9 M, 2.0 M, 2.1 M, 2.2 M, 2.3 M, 2.4 M, 2.5 M, 2.6 M, 2.7 M, 2.8 M, 2.9 M, or 3.0 M. In some embodiments, the concentrated salt solution comprises one or more metal halide salts. In some embodiments, the metal halide salt is selected from the group consisting of LiF, LiCl, LiBr, LiI, NaF, NaCl, NaBr, NaI, KF, KCl, KBr, KI, RbF, RbCl, RbBr, RbI, CsF, CsCl, CsBr, CsI, BeF₂, BeCl₂, BeBr₂, BeI₂, MgF₂, MgCl₂, MgBr₂, MgI₂, CaF₂, CaCl₂, CaBr₂, CaI₂, SrF₂, SrCl₂, SrBr₂, SrI₂, BaF₂, BaCl₂, BaBr₂, BaI₂, and mixtures thereof. In some embodiments, the concentrated salt solution comprises KCl and/or KI. In some embodiments, the tissue is concurrently exposed to KCl and KI, and the concentrated salt solution comprises 0.6 M KCl and 1.0 M KI. In varying embodiments, the tissue is sequentially exposed to KCl and KI, *e.g.,* first 0.6 M KCl and then 1.0 M KI or first 1.0 M KI and then 0.6 M KCl. The tissue can be washed in an isotonic solution between exposure to the high concentration salt solutions. As appropriate, the high concentration salt solution may further comprise a protease inhibitor and one or more antimicrobial agents, *e.g.,* 0.5 mM AEBSF (4-(2-Aminoethyl) benzenesulfonyl fluoride hydrochloride) ("PEFABLOC™") and 1% Antibiotic Antimycotic Solution (AAS).

In varying embodiments, the muscle tissue is submerged in or perfused with the concentrated salt solution. In some embodiments, the muscle tissue is washed or rinsed with the concentrated salt solution. In some embodiments, the relaxing solution, described above, comprises one or more salts at a high concentration. In varying embodiments, the muscle tissue is contacted with the concentrated salt solution after exposure to the relaxing solution.

In some embodiments, sarcomeric constituents are not detectable in the muscle tissue. In some embodiments, at least about 90% of the sarcomeric constituents are removed, *e.g.,* at least about 93%, 95%, 97%, 98%, 99% or all (100%) sarcomeric constituents are removed.

### 4. Tissue Scaffolds and/or Decellularized Extracellular Matrix (ECM)

Removing antigen components from tissue by sequentially immersing the tissue in separate solutions for depolymerizing and/or destabilizing one or more cytoskeletal components, solubilizing water soluble antigen components and for solubilizing lipid soluble antigen components produces tissue scaffolds and/or decellularized extracellular matrix (ECM) with biophysical and biochemical properties substantially the same as the tissue before it is subject to antigen removal. The produced tissue scaffolds and/or decellularized extracellular matrix (ECM) moreover have low residual antigenicity (*e.g.,* to an allogeneic or xenogeneic host), and do not elicit significant or destructive immune responses by the host against the tissue scaffolds and/or decellularized extracellular matrix (ECM). The tissue scaffolds and/or decellularized extracellular matrix (ECM) produced according to the methods described herein are also non-toxic to live cells and suitable for repopulation and/or recellularization with live cells, *e.g.,* that are allogeneic or xenogeneic to the tissue scaffolds and/or decellularized extracellular matrix (ECM).

With respect to residual antigenicity, the tissue scaffolds and/or decellularized extracellular matrix (ECM) that have been subject to sequential solubilization procedures for removal of water-soluble and lipid-soluble antigenic components are substantially depleted of water soluble and lipid soluble antigen components. In various aspects, at least about 80%, for example, at least about 85%, 90%, 93%, 95%, 97%, 99%, or more, of the water soluble antigens are removed from the tissue to produce the present tissue scaffolds and/or decellularized extracellular matrix (ECM) (*e.g.,* the residual antigenicity of water-soluble antigens is less than about 20%, *e.g,* less than about 15%, 10%, 7%, 5%, 3%, 1%, or less). In some aspects, at least about 60%, for example, at least about 65%, 70%, 75%, 80%, 85%, 90%, 93%, 95%, 97%, 99%, or more, of the lipid soluble antigens are removed from the tissue to produce the present tissue scaffolds and/or decellularized extracellular matrix (ECM) (*e.g.,* the residual antigenicity of lipid-soluble antigens is less than about 40%, *e.g,* less than about 35%, 30%, 25%, 20%, 15%, 10%, 7%, 5%, 3%, 1%, or less). In various aspects, the water-soluble and/or lipid soluble antigen components are not detectable in the produced tissue scaffolds and/or decellularized extracellular matrix (ECM). The presence and/or amount of water-soluble and/or lipid soluble antigen components remaining in the tissue scaffolds and/or decellularized extracellular matrix (ECM) after performing antigen removal can be detected using methods known in the art, *e.g., in vitro* and/or *in vivo* immunoassays, Western blotting, ELISA, gel electrophoresis, lymphocyte proliferation and/or migration assays, *etc.*

For determination of residual antigenicity *in vivo,* tissue scaffolds and/or decellularized ECM produced according to the methods described herein (*e.g*., repopulated or not repopulated with live cells) can be implanted subcutaneously in a host that is allogeneic or xenogeneic to the tissue scaffold or decellularized ECM. Post-implantation development of scaffold calcification, innate (histologic assessment), humoral (biomaterial specific IgG positivity, C4d deposition) and cell-mediated (IHC for CD4/CD8/Treg subtyping, lymphocyte proliferation and migration assays) immune responses can be assessed and compared to both positive (native tissue) and clinically relevant negative control tissue (*e.g.,* native allograft heart muscle tissue/patch, glutaraldehyde-fixed tissue). Such assays are known in the art. In various aspects, the produced tissue scaffolds and/or decellularized extracellular matrix (ECM) do not elicit a detectable immune response, *e.g.,* by lymphocytes allogeneic or xenogeneic to the tissue scaffold and/or ECM as measured in *in vitro* and/or *in vivo* assays. In various aspects, the produced tissue scaffolds and/or decellularized extracellular matrix (ECM) elicit a detectable but insignificant and/or nondestructive immune response, *e.g.,* by lymphocytes allogeneic or xenogeneic to the tissue scaffold and/or ECM as measured in *in vitro* or *in vivo* assays. In various aspects, the produced tissue scaffolds and/or decellularized extracellular matrix (ECM) have a residual antigenicity and elicit an immune response that is equal to or less than the immune response of a negative control tissue (e.g., native allograft heart muscle tissue/patch, glutaraldehyde-fixed tissue).

With respect to biophysical properties, the tissue scaffolds and/or decellularized extracellular matrix (ECM) produced according to the present methods retain structure and strength that are not significantly different from or are substantially the same as the tissue before it is subject to antigen removal, *e.g.,* by sequential solubilization of water-soluble and lipid-soluble antigen components. For example, the matrix morphology or structural integrity of the tissue scaffolds and/or decellularized extracellular matrix (ECM) is substantially intact-not significantly collapsed, degraded, shrunken, buckled, twisted or otherwise deformed. The size, *e.g.,* length, width, thickness and/or volume, of the tissue scaffolds and/or decellularized extracellular matrix (ECM) are not significantly different from or are substantially the same as the tissue before it is subject to antigen removal. The structure of the tissue scaffolds and/or decellularized ECM produced according to the present methods retain a size, shape and structural integrity that is not significantly different from or is substantially the same as the tissue before it is subject to antigen removal. The structural integrity of the produced tissue scaffolds and/or decellularized ECM can be determined using any method in the art, *e.g.,* including histology and electron microscopy (*e.g*., transition electron and/or scanning electron microscopy). *See, e.g.,* Williams, et al., Acta Biomater. (2009) 5(4):993-1005; Zou and Zhang, J Surg Res. (2011) PMID 21571306. Histology techniques can be utilized to assess tissue morphology, residual nuclei counts (*e.g*., Hematoxylin and Eosin (H&E) staining), and gross ECM structure (*e.g*., Verhoeff-van Gieson staining). Collagen fiber orientation, mean fiber diameter and percent volume can be evaluated using electron microscopy. The tissue scaffolds and/or decellularized extracellular matrix produced according to the present methods moreover retain tensile properties, *e.g.,* as measured by Young's modulus, tensile stress, and tensile strain parameters, that are not significantly different from or are substantially the same as the tissue before it is subject to antigen removal. Methods for determining biophysical properties of a tissue, before and after antigen removal, are known in the art and find use. For example, the size and/or volume of a sample of tissue can be determined before and after antigen removal, *e.g.,* using a calipers. Methods for determining Young's modulus, tensile stress, and/or tensile strain of a tissue are also known in the art and find use. Illustrative methods are described, *e.g.,* in Wong, et al, Biomaterials. (2011) 32:8129-8138; Ling Y. AMP J Tech. (1996) 5:37-48; Sheridan, et al., J Mech Behav Biomed Mater. (2012) 8:58-70; Williams, et al., Acta Biomater. (2009) 5(4):993-1005; Zou and Zhang, J Surg Res. (2011) PMID 21571306; and Petersen, et al., Cells Tissues Organs. (2012) 195(3):222-31.

With respect to biochemical properties, the tissue scaffolds and/or decellularized extracellular matrix (ECM) produced according to the present methods retain quantitative biochemical properties that are not significantly different from or are substantially the same as the tissue before it is subject to antigen removal, *e.g.,* by sequential solubilization of water-soluble and lipid-soluble antigen components. For example, quantitative content (*e.g.,* amounts and/or ratios) of water, elastin, collagen, glycosaminoglycans and/or proteoglycans is not significantly different or is substantially the same in tissues before and after antigen removal by the present methods. Methods for quantitative determination of biochemical properties of a tissue sample, *e.g.,* content of water, elastin, collagen, glycosaminoglycans (GAG), proteoglycans, and/or other ECM components, are known in the art and find use. Illustrative methods are described, *e.g.,* in Wong, et al, Biomaterials. (2011) 32:8129-8138; Williams, et al., Acta Biomater. (2009) 5(4):993-1005; Petersen, et al., Cells Tissues Organs. (2012) 195(3):222-31 and Woessner. Arch Biochem Biophys (1961) 93:440e7. Assay kits for determining the content of collagen, elastin, proteoglycans and/or GAG find use and are commercially available, *e.g.,* from Biocolor Ltd. (on the internet at biocolor.co.uk), Worthington Biochemical Corp. (on the internet at worthington-biochem.com), Sigma-Aldrich (on the internet at sigmaaldrich.com), Quickzyme (on the internet at quickzyme.com), Kamiya Biomedical Company (on the internet at kamiyabiomedical.com) and Astarte Biologics (on the internet at astartebio.com).

With respect to live cell repopulation and/or recellularization, the tissue scaffolds and/or decellularized extracellular matrix (ECM) produced according to the present methods are suitable for repopulation or recellularization with live cells. Generally, the tissue scaffolds and/or decellularized ECM do not contain toxic contaminants or residue from the antigen removal process that are toxic to live cells and impede the ability of live cells to repopulate or recellularize the tissue scaffold or ECM. For example, in various aspects, the tissues subject to sequential solubilization of water-soluble and lipid-soluble antigens for antigen removal are not contacted with sodium dodecyl sulfate (SDS). In various aspects, the tissue scaffolds and/or decellularized extracellular matrix (ECM) produced according to the present methods are substantially repopulated with live cells, *e.g.,* at least about 60%, 65%, 70%, 75%. 80%, 85%, 90%, 95%, 99%, or more, of capacity repopulated or recellularized. The extent of recellularization can be determined using any method in the art. Illustrative assays for assessing recellularizaton capacity include without limitation toxicity (*e.g.,* LDH), adhesion (*e.g.,* histology), viability (*e.g.,* Live/Dead), migration (*e.g.,* hMSC tissue invasion on histology and immunohistochemistry (IHC)), and proliferation (*e.g.,* proliferating cell nuclear antigen antibody (PCNA)) assays. As appropriate and depending on tissue to be recellularized and cell/donor availability, the tissue scaffolds and/or decellularized ECM can be repopulated and/or recellularized with cells autologous to the recipient of the tissue; allogeneic to the recipient of the tissue; cells of the same tissue type as the tissue to be transplanted into the recipient; mesenchymal stem cells; and mixtures thereof.

Tissue scaffolds and /or decellularized ECM produced according to the methods described herein have application in processing and implantation of bioprostheses, biomaterials, or xenogeneic tissues (xenografts) including, *e.g.,* heart valves, vascular conduits, arteries, veins, skin, dermis, ligaments, tendons, bone, cartilage, muscle, ureter, urinary bladder, liver, heart, and other organs; or processing and transplantation of fresh, preserved, or banked allogeneic tissues (allografts) including vessels, vascular conduits, arteries, veins heart valves, skin, dermis ligaments, tendons, bone, cartilage, muscle, ureter, urinary bladder, liver, heart, or other organs; or the development of natural biological matrices for tissue-engineered tissues and organs including heart valves, vessels, skin, dermis, ligaments, bone, cartilage, muscle, ureter, urinary bladder, liver, heart, or other organs.

In various aspects, the tissue scaffolds and/or decellularized ECM can be used as implants, tissue fillers, burn dressings, wound dressings, blood vessel grafts, blood vessel replacements, and the like. Medical graft materials comprising the tissue scaffolds and/or decellularized ECM produced by sequential antigen removal methods can be used in the repair or reconstruction of tissues such as nervous tissue, dermal tissue (ex: in wound care), cardiovascular tissue (including vascular and cardiac), pericardial tissue, muscle tissue, bladder tissue, ocular tissue, periodontal tissue, bone, connective tissue (tendons, ligaments), and the like. Medical graft materials of the disclosure can also be used in conjunction with one or more secondary components to construct a medical device (*e.g.,* a balloon-expandable or self-expanding stent).

Another application of a method according to the invention is to mitigate, reduce, inhibit and/or prevent immune rejection of transplanted tissues/organs between individuals within a species (i.e., allografts) or between species (i.e., xenografts) by the removal of antigens from the tissue/organ.

If the tissue scaffold and/or decellularized ECM is sterilized following the antigen removal procedure, it should have a shelf life of at least 1 year, or more.

### 5. Kits

The disclosure further provides kits comprising tissue scaffolds and/or decellularized ECM, *e.g.,* as described herein and/or produced according to the methods described herein. The kits may further comprise instructions for repopulation or recellularization of the tissue scaffolds and/or decellularized ECM with live cells. Generally, tissue scaffolds and/or decellularized ECM provided in such kits are sterilized and ready for transplantation into a host.

Also provided are kits for producing tissue scaffolds and/or decellularized ECM substantially depleted of antigenic components and suitable for live cell repopulation or recellularization. In varying aspects, the kits comprise a first container comprising a solution for depolymerizing one or more cytoskeletal components, a second container comprising a solution for solubilizing water-soluble antigens and a third container comprising a solution for solubilizing lipid-soluble antigens. The aspects of the solutions for depolymerizing one or more cytoskeletal components, for solubilizing water-soluble antigens and for solubilizing lipid-soluble antigens are described above and herein. In varying aspects, the kits may further comprise a container comprising a relaxing solution and a container comprising a concentrated salt solution. The aspects of the relaxing solution and the concentrated salt solution are described above and herein. The kits may further comprise instructions for removing antigenic components from a tissue, *e.g.,* a muscle tissue. In various aspects, the kits also comprise a control tissue scaffold and/or decellularized ECM, *e.g.,* that has been processed employing sequential antigen removal and produced according to methods described herein.

### EXAMPLES

The following examples are offered to illustrate, but not to limit the claimed invention.

### Example 1

### Antigen Removal from Cardiac Muscle Tissue

### MATERIALS AND METHODS

*Myocardial patch (MP) isolation.* Whole hearts were isolated from Fischer rats (CDF®,CRL, Kingston, NY) being euthanized from other non-cardiac studies, perfused with heparin via antegrade coronary perfusion and stored in storage solution (DMEM with 15% DMSO v/v) at -80°C. Hearts are defrosted at room temperature and blood removed by antegrade coronary perfusion with 4°C heparinized PBS (10IU/ml). MPs are prepared as follows: The left ventricle (LV) is isolated and two 4mm thick short axis LV slices cut from base to apex. Each LV slice is cut longitudinally through the LV free wall between the papillary muscles. A 3.5 mm biopsy punch (Miltex) is utilized to cut adjacent cylindrical pieces of LV tissue. This approach allows for the generation of 6 identical LV myocardial patches (MP) from each LV slice (total of 12 MPs per heart). MPs are placed in Relaxing Solution comprising of 120 mM KCl, 4 mM MgCl₂, 4 mM EGTA, 20 mM TrisHCl pH 7.5, 5.88 mM NaATP, 0.5mM Pefabloc, 1% Antibiotic Antimycotic Solution (AAS;Sigma) on ice upon isolation until further processing. MPs are patted dry twice and their wet weights recorded (approximately 20 mg per patch).

*Generation of mouse anti-rat LV anti-serum.* Murine sera generation was conducted following UC Davis institutional procedures and was covered by IACUC #15064. Isolated adult Fischer rat LV's (n=6) were homogenized and injected subcutaneously into C57BL/6 mice (n=6) on days 0, 14, 28, 42, 56 and 72. Serum was collected at days 0, 14, 28, 42, 56, 72, and 84, and stored at -80°C.

*Antigen removal (AR) procedure.* All steps of the AR protocol are performed at 125 rpm and at RT or 4°C unless otherwise stated. AR procedure for Water-Soluble antigens (WSA) and Lipid-Soluble antigens (LSA) involves incubating MPs in any given AR additive such as the examples shown in (Table 1) for 1-3 days, depending on the additive used. Anatomically adjacent MP pieces are subjected to 1 min incubations and serve as negative AR controls. Following nucleic acid digestion for 24 h and washout for 48 h, MPs following AR (designated as MP-AR) are stored in 15% DMSO in DMEM at -80°C.

**TABLE 1**

| **ANTIGEN REMOVAL ADDITIVES FOR EACH PROTEIN CLASS AND LEVELS USED** | | |
|---|---|---|
| **AR Step** | **Additive** | **[Function/Levels]** |
| Water-Soluble proteins | BARB (Basic AR Buffer); 0.5 mM Pefabloc (Roche) and 1% AAS in 10 mM Tris-HCl, pH 8.0 | Hypotonic solution containing protease inhibitors and antibiotic/antimycotic agent; Cell lysis |
| Water-Soluble proteins | optSARB (optimized Solubilizing Antigen Removal Buffer; 100 mM DTT, 2 mM MgCl₂, 100 mM KCl in BARB) | BARB corrected for isotonic/physiologic salt concentration; protein/antigen solubilization with use of reducing reagent (DTT) |
| Actin Depol ymerizati on | Cytochalasin D in organic solvent (such as DMSO) or high glucose DMEM | 0, 100nM, 1µM, 10µM |
| Actin Depol ymerizati on | Latrunculin B in high glucose DMEM | 0, 50nM, 100nM, 200nM |
| Actin Depol ymerizati on | Mycalolide B in organic solvent (such as DMSO) or high glucose DMEM | 0, 100nM, 1µM, 10µM |
| Actin Depol ymerizati on | Swinholide A in organic solvent (such as DMSO) | 0, 100nM, 1µM, 10µM |
| Cardiac Myosin solubilization | KCl in aqueous buffer containing protease inhibitors and antibiotics | 400mM, 500mM, 600mM, 800mM |
| Cardiac Titin solubilization | KI in aqueous buffer containing protease inhibitors and antibiotics | 400mM, 600mM, 800mM, 1M |
| Lipid-Soluble proteins | Amidosulfobetaine 14 (ASB-14) | Sulfobetaine detergent. 0, 1%, 2%, 3%, 4%, 5% |
| Lipid-Soluble proteins | ASB-16 | Sulfobetaine detergent. 0, 0.05%, 0.1%, 1%, 2% |
| Lipid-Soluble proteins | Sulfobetaine 3-12 (SB 3-12) | Sulfobetaine Detergent. 0, 0.1%, 0.2%, 0.5%, 2% |
| Lipid-Soluble proteins | SB 3-14 | Sulfobetaine Detergent. 0, 0.1%, 1%, 2%, 4%, 5% |
| [Decellularization/ Literature control] | 1% SDS (w/v) | Strong anionic detergent, literature control |

*Example of Water-Soluble and Lipid-Soluble Antigen Removal on a Myocardial Patch.* All steps are performed at 125 rpm at RT unless otherwise stated. Following MP isolation and recording of wet weights as described above, MPs are incubated in relaxing solution for 2x15 min at 125 rpm, at 4°C. MPs are then incubated in the lipid-soluble antigen removal solution comprising of 2% ASB in optimized Standard Antigen Removal Buffer (optSARB; 0.5 mM Pefabloc and 1% AAS in 10 mM Tris-HCl, pH 8.0, 100 mM DTT, 2 mM MgCl₂, 100 mM KCl) for 2 days at 4°C, with one addition of fresh solution after 24 hrs. MPs are then washed 2x15 min in optSARB, before proceeding with sarcomere disassembly using actin depolymerization followed by solubilization of sarcomeric components and associated water-soluble antigens. Specifically, MPs are incubated for 2 h at 37°C in 50 nM Latrunculin B in high-glucose DMEM, washed 2x15 min in optSARB, incubated for 2 h in 0.6 M KCl containing 0.5 mM Pefabloc and 1% AAS, washed for 2x15 min in optSARB, incubated for 2 h in 1.0 M KI containing 0.5 mM Pefabloc and 1% AAS, after which they are left in optSARB at 4°C overnight.

The following day KCl and KI incubations are repeated as described above, with 2x15 min optSARB washes in between them, followed by optSARB wash overnight at 4°C. The next day fresh optSARB is applied to the MP-AR scaffolds for 24 h at 4°C. Following this, MP-AR scaffolds are incubated for 24 h at 4°C with Nuclease solution (2.5 Kunitz units/mL DNAse I, 7.5 Kunitz units/mL RNAse A, 1% AAS, 0.15 M NaCl, 5 mM MgCl₂ - 6H₂O in 10 mM Tris-HCl, pH 7.6). Then, they are washed for 48 h in washing solution (0.5 mM Pefabloc and 1% AAS in 1x TBS) which is changed to fresh solution 1/day and lastly, the scaffolds are stored at 80°C in 15% v/v DMSO in high glucose DMEM storage solution.

*Assessment of residual antigenicity in MP-AR samples.* MP-AR samples and controls are minced and residual proteins extracted. Extracted proteins are subjected to one-dimensional electrophoresis (1-DE) and Western blot (WB) probed with mouse anti-rat native LV poly-polyclonal anti-serum diluted 1:100 and assessed for IgG positivity with 1:10,000 hrp-conjugated anti-mouse light chain specific secondary antibody (Jackson Immunoresearch). Western blots are imaged using FluorChem Xplor CCD bioimaging system and AlphaView image acquisition and analysis software (Alpha Innotech Corp). Densitometry is used to quantify banding pattern intensity. Residual antigenicity of MP-AR is defined as the ratio of banding intensity for extracts following 2 d of AR compared the 1 min AR controls. Overall residual antigenicity for each AR protocol is calculated with respect to the level of antigens present in negative control. *See,* Table 1 and Figures 1-2.

### Example 2

### Implant Data

One week after subpannicular implantation, a non-specific inflammatory response following the surgery was observed toward all bovine pericardium (BP) scaffolds. No dramatic differences in this response were observed between treatment groups (Fig. 3).

Six weeks after subpannicular implantation, the *in vivo* immune response of New Zealand White rabbits towards BP following stepwise, solubilization-based antigen removal (BP-AR) using opt SARB, followed by ASB-14 was markedly less than that towards native BP; BP following one-step, solubilization-based antigen removal (opt SARB); BP decellularized with 1% (w/v) SDS; and even the current clinically accepted gold standard glutaraldehyde-fixed BP (Fig. 4).

A minimal amount of small mononuclear cells (MNCs) were observed in the subdermal, subpannicular, and peri-scaffold regions associated with ASB-14 treated BP-AR. A mild level of small MNCs is associated with SDS-decellularized BP. A moderate amount of small MNCs were found with fixed BP or native BP. A severe level of small MNCs, including formation of lymphoid follicles, was associated with opt SARB-treated BP-AR. Stepwise antigen removal using ASB-14 decreased the amount of MNCs in the peri-scaffold region compared to native BP, opt SARB-treated BP-AR, SDS-decellularized BP, or fixed BP.

Little to no fibrous encapsulation was seen with ASB-14 treated BP-AR, opt SARB-treated BP-AR, or native BP. Conversely, notable fibrous encapsulation was associated with SDS-decellularized BP or fixed BP. Stepwise antigen removal using ASB-14 was associated with less fibrous encapsulation than SDS-decellularized BP or fixed BP.

Marked cellular ingrowth was observed with ASB-14 treated BP-AR or opt SARB-treated BP-AR. However, minimal cellular ingrowth was associated with SDS-decellularized BP or fixed BP. Stepwise antigen removal using ASB-14 supported robust cellular repopulation of BP-AR.

## Claims

1. A method of removing immunogenic antigens from an intact tissue, comprising:
a) contacting the intact tissue with one or more actin depolymerization agents, and/or one or more microtubule depolymerization or microtubule destabilizing agents;
b) solubilizing water-soluble antigens in the tissue, wherein the water-soluble antigens are solubilized in a first solution comprising a first buffering agent, a reducing agent, a protease inhibitor, and one or more metal halide salts, wherein the first solution does not comprise an amphiphile;
c) separating the tissue from the solubilized water-soluble antigens;
d) solubilizing lipid-soluble antigens in the tissue, wherein the lipid-soluble antigens are solubilized in a second solution comprising a second buffering agent, a reducing agent, a protease inhibitor, one or more salts suitable for maintaining protein solubility and an amphiphile; and
e) separating the tissue from the solubilized lipid-soluble antigens; thereby removing immunogenic antigens from the tissue, wherein the method yields a substantially intact extracellular matrix (ECM) compatible with viable cell repopulation.

2. The method of claim 1, wherein;
a) one or more of the steps are repeated;
b) the immunogenic antigens are selected from the group consisting of protein antigens, lipid antigens and carbohydrate antigens;
c) the method does not comprise contacting the tissue with a detergent selected from the group consisting of sodium dodecyl sulfate, Triton-X-100, Triton-X-114, Triton-X-200 and sodium deoxycholate; and/or
d) the method does not comprise contacting the tissue with a protease.

3. The method of any one of claims 1 to 2, wherein:
a) the one or more actin depolymerization agents are selected from the group consisting of Cytochalasin A, Cytochalasin B, Cytochalasin C, Cytochalasin D, Cytochalasin E, Cytochalasin F, Cytochalasin G, Cytochalasin H, Cytochalasin I, Cytochalasin J, Latrunculin A, Latrunculin B, Swinholide A, Misakinolide A, Bistheonelide A, Scytophycin A, Scytophycin B, Scytophycin D, Scytophycin E, 19-0-Demethylscytophycin C, 6-Hydroxyscytophycin B, 6-Hydroxy-7-o-methylscytophycin E and tolytoxin, Mycalolide A, Mycalolide B, Mycalolide C, secomycalolide A and 30-hydroxymycalolide A, Halichondramide, (19Z)-halichondramide, kabiramides B, kabiramides C, kabiramides D, kabiramides G, kabiramides J, kabiramides K, ulapualide A, jaspamide, Dihydrohalichondramide, Aplyronine A, Aplyronine B, Aplyronine C, Pectenotoxin 2, Pectenotoxin 6, and Migrastatin, and/or
b) the one or more microtubule depolymerization or destabilizing agents are selected from the group consisting of colchicine, colcemid, vinblastine, vincristine, myoseverin, nocodazole, podophyllotoxin, polygamain and taxol.

4. The method of claim 1, wherein;
a) the first buffering agent maintains a pH in the range of about 4-11;
b) the one or more metal halide salts are selected from the group consisting of LiF, LiCl, LiBr, LiI, NaF, NaCl, NaBr, NaI, KF, KCl, KBr, KI, RbF, RbCl, RbBr, RbI, CsF, CsCl, CsBr, CsI, BeF₂, BeCl₂, BeBr₂, BeI₂, MgF₂, MgCl₂, MgBr₂, MgI₂, CaF₂, CaCl₂, CaBr₂, CaI₂, SrF₂, SrCl₂, SrBr₂, SrI₂, BaF₂, BaCl₂, BaBr₂, BaI₂, and mixtures thereof;
c) the reducing agent is selected from the group consisting of Tributylphosphine (TBP), beta mercaptoethanol, 2 mercaptoethylamine, tris(2-carboxyethyl)phosphine (TCEP), cysteine-HCl, and dithiothreitol (DTT);
d) the water-soluble antigens are solubilized in a solution that further comprises a chelation agent; and/or
e) the water-soluble antigens are solubilized in a first solution comprising Tris-HCl, dithiothreitol (DTT), a first protease inhibitor, and KCl.

5. The method of any one of claims 1 to 4, wherein the amphiphile in the second solution is a sulfobetaine.

6. The method of claim 5, wherein the sulfobetaine is selected from the group consisting of 3-[N,N-Dimethyl(3-myristoylaminopropyl)ammonio]propanesulfonate (amidosulfobetaine-14; ASB-14); amidosulfobetaine-16 (ASB-16); 4-n-Octylbenzoylamido-propyl-dimethylammonio sulfobetaine (ASB-C8Ø); 3-(N,N-Dimethyloctylammonio) propanesulfonate inner salt (SB3-8); N-Decyl-N,N-dimethyl-3-ammonio-1-propanesulfonate (SB 3-10), N-Decyl-N,N-dimethyl-3-ammonio-1-propanesulfonate (SB 3-12), N-Tetradecyl-N,N-dimethyl-3-ammonio-1-propanesulfonate (SB 3-14); 3-(N,N-Dimethylpalmitylammonio) propanesulfonate (SB3-16); 3-(N,N-Dimethyloctadecylammonio) propanesulfonate (SB3-18); 3-(1-Pyridinio)-1-propanesulfonate (NDSB-201); 3-(Benzyldimethylammonio) propanesulfonate (NDSB-256); NDSB-211, NDSB-195, NDSB-221; 3-[(3-Cholamidopropyl)dimethylammonio]-1-propanesulfonate (CHAPS), 3-[(3-Cholamidopropyl)dimethylammonio]-2-hydroxy-1-propanesulfonate (CHAPSO), and mixtures thereof.

7. The method of any one of claims 1 to 6, wherein the lipid-soluble antigens are solubilized in a solution comprising Tris-HCl, dithiothreitol (DTT), a protease inhibitor, KCl and ASB-14.

8. The method of claim 7, further comprising repopulating the ECM with live cells.

9. The method of any one of claims 1 to 8, wherein the tissue is:
a) epithelial tissue, endothelial tissue, muscle tissue, or connective tissue;
b) selected from the group consisting of cardiac muscle tissue, striated or skeletal muscle tissue, or smooth muscle tissue, heart, pericardium, heart valve, vessel, vascular conduit, artery, vein, skin, dermis, pericardium, dura, intestinal submucosa, ligament, tendon, bone, cartilage, ureter, urinary bladder, kidney, skin, lung, liver, and umbilical cord;
c) muscle tissue;
d) muscle tissue selected from the group consisting of cardiac muscle tissue, striated or skeletal muscle tissue and smooth muscle tissue; and/or
e) a part of an intact organ.

10. The method of any one of claims 1 to 9, wherein the tissue is intact tissue, the method comprising:
a) solubilizing water-soluble antigens in the tissue by contacting the tissue with the first solution comprising Tris-HCl, dithiothreitol (DTT), the protease inhibitor, and KCl, wherein the first solution does not comprise an amphiphile, such that the water-soluble antigens are solubilized in the first solution;
b) separating the tissue from the solubilized water-soluble antigens in the first solution;
c) solubilizing lipid-soluble antigens in the tissue by contacting the tissue with a second solution comprising Tris-HCl, dithiothreitol (DTT), the protease inhibitor, KCl and ASB 14, such that the lipid-soluble antigens are solubilized in the second solution; and
d) separating the tissue from the solubilized lipid-soluble antigens in the second solution; thereby removing immunogenic antigens from the intact tissue.

11. The method of claim 1, wherein the tissue is muscle tissue, comprising:
a) relaxing the muscle tissue in a solution comprising an energy source molecule;
b) contacting the muscle tissue with one or more actin depolymerization agents, and/or one or more microtubule depolymerization or microtubule destabilizing agents;
c) contacting the muscle tissue with a concentrated salt solution;
d) solubilizing water-soluble antigens in the tissue as in claim 1;
e) separating the muscle tissue from the solubilized water-soluble antigens;
f) solubilizing lipid-soluble antigens in the tissue as in claim 1; and
g) separating the muscle tissue from the solubilized lipid-soluble antigens;
thereby removing immunogenic antigens from the muscle tissue.

12. The method of claim 11, wherein:
a) one or more of the steps are repeated;
b) wherein the steps are performed in an order selected from the group consisting of:
i) a), b), c), d), e), f) and g);
ii) a), f), g), b), c), d) and e);
iii) a), d), e), b), c), f) and g);
iv) a), d), e), f), g), b) and c);
v) a), b), c), d), e), f), g), b), c), and e); and
vi) a), f), g), b), c), d), e), f), and g);
c) the muscle tissue is cardiac muscle tissue, striated or skeletal muscle tissue, or smooth muscle tissue;
d) the method comprises the step of relaxing the muscle tissue prior to contacting the tissue with one or more cytoskeletal destabilizing agents;
and/or
e) sarcomeric constituents are not detectable in the muscle tissue.

13. The method of claim 11, wherein the energy source molecule is:
a) selected from the group consisting of a nucleotide 5'-triphosphate (NTP), adenosine, inosine, aspartate, glutamate, creatine phosphate, a Kreb's cycle precursor or intermediate, glucose, and dextrose;
b) a nucleotide 5'-triphosphate (NTP) selected from the group consisting of adenosine 5'-triphosphate (ATP), inosine 5'-triphosphate (ITP), guanidine 5'-triphosphate (GTP), cytidine 5'-triphosphate (CTP), and uridine 5'-triphosphate (UTP); and/or
c) a precursor of adenosine 5'-triphosphate (ATP).

14. The method of any one of claims 11 to 13, wherein the relaxing solution further comprises:
a) a calcium ion chelating agent;
b) a permeabilization agent; and/or
c) a protease inhibitor and an antimicrobial agent.

15. The method of any one of claims 11 to 14, wherein the concentrated salt solution comprises:
a) one or more salts in a concentration range from about 0.5 M to about 3.0 M; and/or
b) one or more halide salts.

## Patentansprüche

1. Verfahren zum Entfernen immunogener Antigene aus einem intakten Gewebe, umfassend:
a) Inberührungbringen des intakten Gewebes mit einem oder mehreren Actin-Depolymerisationsmitteln und/oder einem oder mehreren Mikrotubulus-Depolymerisations- oder Mikrotubulus-destabilisierenden Mitteln;
b) Auflösen von wasserlöslichen Antigenen in dem Gewebe, wobei die wasserlöslichen Antigene in einer ersten Lösung aufgelöst werden, die ein erstes Puffermittel, ein Reduzierungsmittel, einen Proteasehemmer und ein oder mehrere Halogenidsalze umfasst, wobei die erste Lösung kein Amphiphil umfasst;
c) Trennen des Gewebes von den aufgelösten wasserlöslichen Antigenen;
d) Auflösen von lipidlöslichen Antigenen in dem Gewebe, wobei die lipidlöslichen Antigene in einer zweiten Lösung aufgelöst werden, die ein zweites Puffermittel, ein Reduzierungsmittel, einen Proteasehemmer und ein oder mehrere Salze, die zum Erhalten der Proteinlöslichkeit geeignet sind, und ein Amphiphil umfasst; und
e) Trennen des Gewebes von den aufgelösten lipidlöslichen Antigenen; wobei dadurch immunogene Antigene aus dem Gewebe entfernt werden, wobei das Verfahren eine im Wesentlichen intakte extrazelluläre Matrix (ECM) ergibt, die mit lebensfähiger Zellrepopulation vereinbar ist.

2. Verfahren nach Anspruch 1, wobei;
a) einer oder mehrere der Schritte wiederholt werden;
b) die immunogenen Antigene aus der Gruppe ausgewählt sind, die aus Proteinantigenen, Lipidantigenen und Kohlenhydratantigenen besteht;
c) das Verfahren nicht das Inberührungbringen des Gewebes mit einem Netzmittel umfasst, das aus der Gruppe ausgewählt ist, die aus Natriumdodecylsulfat, Triton-X-100, Triton-X-114, Triton-X-200 und Natriumdeoxycholat besteht; und/oder
d) das Verfahren nicht das Inberührungbringen des Gewebes mit einer Protease umfasst.

3. Verfahren nach einem der Ansprüche 1 bis 2, wobei:
a) das eine oder die mehreren Actin-Depolymerisationsmittel aus der Gruppe ausgewählt sind, die aus Cytochalasin A, Cytochalasin B, Cytochalasin C, Cytochalasin D, Cytochalasin E, Cytochalasin F, Cytochalasin G, Cytochalasin H, Cytochalasin I, Cytochalasin J, Latrunculin A, Latrunculin B, Swinholid A, Misakinolid A, Bistheonelid A, Scytophycin A, Scytophycin B, Scytophycin D, Scytophycin E, 19-0-Demethylscytophycin C, 6-Hydroxyscytophycin B, 6-Hydroxy-7-o-methylscytophycin E und Tolytoxin, Mycalolid A, Mycalolid B, Mycalolid C, Secomycalolid A und 30-Hydroxymycalolid A, Halichondramid, (19Z)-Halichondramid, Kabiramiden B, Kabiramiden C, Kabiramiden D, Kabiramiden G, Kabiramiden J, Kabiramiden K, Ulapualid A, Jaspamid, Dihydrohalichondramid, Aplyronin A, Aplyronin B, Aplyronin C, Pectenotoxin 2, Pectenotoxin 6 und Migrastatin besteht, und/oder
b) das eine oder die mehreren Mikrotubulus-Depolymerisations- oder - destabilisierende Mittel aus der Gruppe ausgewählt sind, die aus Colchicin, Colcemid, Vinblastin, Vincristin, Myoseverin, Nocodazol, Podophyllotoxin, Polygamain und Taxol besteht.

4. Verfahren nach Anspruch 1, wobei;
a) das erste Puffermittel einen pH-Wert in dem Bereich von etwa 4-11 beibehält;
b) das eine oder die mehreren Halogenidsalze aus der Gruppe ausgewählt sind, die aus LiF, LiCl, LiBr, LiI, NaF, NaCl, NaBr, NaI, KF, KCl, KBr, KI, RbF, RbCl, RbBr, RbI, CsF, CsCl, CsBr, Csl, BeF₂, BeCl₂, BeBr₂, BeI₂, MgF₂, MgCl₂, MgBr₂, MgI₂, CaF₂, CaCl₂, CaBr₂, CaI₂, SrF₂, SrCl₂, SrBr₂, SrI₂, BaF₂, BaCl₂, BaBr₂, BaI₂ und Mischungen davon;
c) das Reduzierungsmittel aus der Gruppe ausgewählt ist, die aus Tributylphosphin (TBP), beta-Mercaptoethanol, 2-Mercaptoenthylamin, tris(2-Carboxyethyl)phosphin (TCEP), Cystein-HCl und Dithiothreitol (DTT) besteht;
d) die wasserlöslichen Antigene in einer Lösung aufgelöst werden, die ferner ein Chelatausbildungsmittel umfasst; und/oder
e) die wasserlöslichen Antigene in einer ersten Lösung aufgelöst werden, die Tris-CHl, Dithiothreitol (DTT), einen ersten Proteasehemmer und KCl umfasst.

5. Verfahren nach einem der Ansprüche 1 bis 4, wobei das Amphiphil in der zweiten Lösung Sulfobetain ist.

6. Verfahren nach Anspruch 5, wobei das Sulfobetain aus der Gruppe ausgewählt ist, die aus 3-[N,N-Dimethyl(3-myristoylaminopropyl)ammonio]propansulfonat(amidosulfobetain-14; ASB-14); Amidosulfobetain-16 (ASB-16); 4-n-Octylbenzoylamido-propyldimethylammoniosulfobetain (ASB-C8Ø); einem inneren Salz von 3-(N,N-Dimethyloctylammonio)propansulfonat (SB3-8); N-Decyl-N,N-dimethyl-3-ammonio-1-propansulfonat (SB 3-10), N-Decyl-N,N-dimethyl-3-ammonio-1-propansulfonat (SB 3-12), N-Tetradecyl-N,N-dimethyl-3-ammonio-1-propansulfonat (SB 3-14); 3-(N,N-Dimethylpalmitylammonio)propansulfonat (SB3-16); 3-(N,N-Dimethyloctadecylammonio)propansulfonat (SB3-18); 3-(1-Pyridinio)-1-propansulfonat (NDSB-201); 3-(Benzyldimethylammonio)propansulfonat (NDSB-256); NDSB-211, NDSB-195, NDSB-221; 3-[(3-Cholamidopropyl)dimethylammonio]-1-propansulfonat (CHAPS), 3-[(3-Cholamidopropyl)dimethylammonio]-2-hydroxy-1-propansulfonat (CHAPSO) und Mischungen davon.

7. Verfahren nach einem der Ansprüche 1 bis 6, wobei die lipidlöslichen Antigene in einer Lösung aufgelöst werden, die Tris-HCl, Dithiothreitol (DTT), einen Proteasehemmer, KCl und ASB-14 umfasst.

8. Verfahren nach Anspruch 7, ferner umfassend die Repopulation der ECM mit lebenden Zellen.

9. Verfahren nach einem der Ansprüche 1 bis 8, wobei das Gewebe ist:
a) Epithelgewebe, Endothelgewebe, Muskelgewebe oder Bindegewebe;
b) aus der Gruppe ausgewählt, die aus Herzmuskelgewebe, striärem oder Skelettmuskelgewebe oder glattem Muskelgewebe, Herz, Pericardium, Herzklappe, Gefäß, vaskulärem Conduit, Arterie, Vene, Haut, Dermis, Pericardium, Dura mater, Darmsubmukosa, Band, Sehne, Knochen, Knorpel, Harnleiter, Harnblase, Niere, Haut, Lunge, Leber und Nabelschnur besteht;
c) Muskelgewebe;
d) Muskelgewebe, das aus der Gruppe ausgewählt ist, die aus Herzmuskelgewebe, striärem oder Skelettmuskelgewebe oder glattem Muskelgewebe besteht; und/oder
e) ein Teil eines intakten Organs.

10. Verfahren nach einem der Ansprüche 1 bis 9, wobei das Gewebe intaktes Gewebe ist, wobei das Verfahren umfasst:
a) Auflösen wasserlöslicher Antigene in dem Gewebe durch das Inberührungbringen des Gewebes mit der ersten Lösung, die Tris-HCl, Dithiothreitol (DTT), den Proteasehemmer und KCl umfasst, wobei die erste Lösung kein Amphiphil umfasst, sodass die wasserlöslichen Antigene in der ersten Lösung aufgelöst werden;
b) Trennen des Gewebes von den wasserlöslichen Antigenen in der ersten Lösung;
c) Auflösen lipidlöslicher Antigene in dem Gewebe durch das Inberührungbringen des Gewebes mit einer zweiten Lösung, die Tris-HCl, Dithiothreitol (DTT), den Proteasehemmer, KCl und ASB 14 umfasst, sodass die lipidlöslichen Antigene in der zweiten Lösung aufgelöst werden; und
d) Trennen des Gewebes von den gelösten lipidlöslichen Antigenen in der zweiten Lösung; wobei dadurch immunogene Antigene aus dem intakten Gewebe entfernt werden.

11. Verfahren nach Anspruch 1, wobei das Gewebe Muskelgewebe ist, umfassend:
a) Entspannen des Muskelgewebes in einer Lösung, die ein Energiequellenmolekül umfasst;
b) Inberührungbringen des Muskelgewebes mit einem oder mehreren Actin-Depolymerisationsmitteln und/oder einem oder mehreren Mikrotubulus-Depolymerisations- oder Mikrotubulus-destabilisierenden Mitteln;
c) Inberührungbringen des Muskelgewebes mit einer konzentrierten Salzlösung;
d) Auflösen von wasserlöslichen Antigenen in dem Gewebe wie in Anspruch 1;
e) Trennen des Muskelgewebes von den aufgelösten wasserlöslichen Antigenen;
f) Auflösen lipidlöslicher Antigene in dem Gewebe wie in Anspruch 1; und
g) Trennen des Muskelgewebes von den aufgelösten lipidlöslichen Antigenen; wobei dadurch immunogene Antigene aus dem Muskelgewebe entfernt werden.

12. Verfahren nach Anspruch 11, wobei:
a) einer oder mehrere der Schritte wiederholt werden;
b) wobei die Schritte in einer Reihenfolge durchgeführt werden, die aus der Gruppe ausgewählt ist, bestehend aus:
i) a), b), c), d), e), f) und g);
ii) a), f), g), b), c), d) und e);
iii) a), d), e), b), c), f) und g);
iv) a), d), e), f), g), b) und c);
v) a), b), c), d), e), f), g), b), c) und e); und
vi) a), f), g), b), c), d), e), f) und g);
c) das Muskelgewebe Herzmuskelgewebe, striäres oder Skelettmuskelgewebe oder glattes Muskelgewebe ist;
d) das Verfahren den Schritt des Entspannens des Muskelgewebes vor dem Inberührungbringen des Gewebes mit einem oder mehreren Cytoskelettdestabilisierungsmitteln umfasst;
e) sarkomere Bestandteile in dem Muskelgewebe nicht nachweisbar sind.

13. Verfahren nach Anspruch 11, wobei das Energiequellenmolekül ist:
a) ausgewählt aus der Gruppe, die aus einem Nukleotid 5'-Triphosphat (NTP), Adenosin, Inosin, Aspartat, Glutamat, Creatinphosphat, einem Krebs-Zyklus-Vorläufer oder - Zwischenprodukt, Glukose und Dextrose besteht;
b) ein Nukleotid 5'-Triophosphat (NTP), das aus der Gruppe ausgewählt ist, die aus Adenosin 5'-Triophosphat (ATP), Inosin 5'-Triophosphat (ITP), Guanidin 5'-Triophosphat (GTP), Cytidin 5'-Triophosphat (CTP) und Uridin 5'-Triophosphat (UTP); und/oder
c) ein Vorläufer von Adenosin 5'-Triophosphat (ATP).

14. Verfahren nach Anspruch 11 bis 13, wobei die Entspannungslösung ferner umfasst:
a) ein Calciumionenchelatausbildungsmittel;
b) ein Permeabilisierungsmittel; und/oder
c) einen Proteasehemmer und ein antimikrobielles Mittel.

15. Verfahren nach einem der Ansprüche 11 bis 14, wobei die konzentrierte Salzlösung umfasst:
a) ein oder mehrere Salze in einem Konzentrationsbereich von etwa 0,5 M bis etwa 3,0 M; und/oder
b) ein oder mehrere Halogenidsalze.

## Revendications

1. Procédé d'élimination d'antigènes immunogènes à partir d'un tissu intact, comprenant :
a) la mise en contact du tissu intact avec un ou plusieurs agents de dépolymérisation d'actine, et/ou un ou plusieurs agents de dépolymérisation de microtubule ou de déstabilisation de microtubule ;
b) la solubilisation d'antigènes hydrosolubles dans le tissu, dans lequel les antigènes hydrosolubles sont solubilisés dans une première solution comprenant un premier agent tampon, un agent réducteur, un inhibiteur de protéase, et un ou plusieurs sels d'halogénure de métal, dans lequel la première solution ne comprend pas d'amphiphile ;
c) la séparation du tissu à partir des antigènes hydrosolubles solubilisés ;
d) la solubilisation d'antigènes liposolubles dans le tissu, dans lequel les antigènes liposolubles sont solubilisés dans une seconde solution comprenant un second agent tampon, un agent réducteur, un inhibiteur de protéase, un ou plusieurs sels appropriés pour maintenir la solubilité protéique et un amphiphile ; et
e) la séparation du tissu à partir des antigènes liposolubles solubilisés ; ainsi éliminant des antigènes immunogènes à partir du tissu, dans lequel le procédé produit une matrice extracellulaire (ECM) sensiblement intacte compatible avec une repopulation cellulaire viable.

2. Procédé selon la revendication 1, dans lequel ;
a) une ou plusieurs des étapes sont répétées ;
b) les antigènes immunogènes sont sélectionnés parmi le groupe constitué de : antigènes protéiques, antigènes lipidiques et antigènes glucidiques ;
c) le procédé ne comprend pas la mise en contact du tissu avec un détergent sélectionné parmi le groupe constitué de : dodécylsulfate de sodium, Triton-X-100, Triton-X-114, Triton-X-200 et désoxycholate de sodium ; et/ou
d) le procédé ne comprend pas la mise en contact du tissu avec une protéase.

3. Procédé selon l'une quelconque des revendications 1 et 2, dans lequel :
a) les un ou plusieurs agents de dépolymérisation d'actine sont sélectionnés parmi le groupe constitué de : cytochalasine A, cytochalasine B, cytochalasine C, cytochalasine D, cytochalasine E, cytochalasine F, cytochalasine G, cytochalasine H, cytochalasine I, cytochalasine J, latrunculine A, latrunculine B, swinholide A, misakinolide A, bisthéonélide A, scytophycine A, scytophycine B, scytophycine D, scytophycine E, 19-0-déméthylscytophycine C, 6-hydroxyscytophycine B, 6-hydroxy-7-o-méthylscytophycine E et tolytoxine, mycalolide A, mycalolide B, mycalolide C, sécomycalolide A et 30-hydroxymycalolide A, halichondramide, (19Z)-halichondramide, kabiramides B, kabiramides C, kabiramides D, kabiramides G, kabiramides J, kabiramides K, ulapualide A, jaspamide, dihydrohalichondramide, aplyronine A, aplyronine B, aplyronine C, pectenotoxine 2, pectenotoxine 6, et migrastatine, et/ou
b) les un ou plusieurs agents de dépolymérisation ou déstabilisation de microtubule sont sélectionnés parmi le groupe constitué de : colchicine, colcémide, vinblastine, vincristine, myosévérine, nocodazole, podophyllotoxine, polygamain et taxol.

4. Procédé selon la revendication 1, dans lequel ;
a) le premier agent tampon maintient un pH dans la plage d'environ 4 à 11 ;
b) les un ou plusieurs sels d'halogénure de métal sont sélectionnés parmi le groupe constitué de : LiF, LiCl, LiBr, Lil, NaF, NaCl, NaBr, NaI, KF, KCl, KBr, KI, RbF, RbCl, RbBr, RbI, CsF, CsCl, CsBr, CsI, BeF₂, BeCl₂, BeBr₂, BeI₂, MgF₂, MgCl₂, MgBr₂, MgI₂, CaF₂, CaCl₂, CaBr₂, CaI₂, SrF₂, SrCl₂, SrBr₂, SrI₂, BaF₂, BaCl₂, BaBr₂, BaI₂, et mélanges de ceux-ci ;
c) l'agent réducteur est sélectionné parmi le groupe constitué de : tributylphosphine (TBP), bêta-mercaptoéthanol, 2 mercaptoéthylamine, tris(2-carboxyéthyl)phosphine (TCEP), cystéine-HCl, et dithiothréitol (DTT) ;
d) les antigènes hydrosolubles sont solubilisés dans une solution qui comprend en outre un agent chélateur ; et/ou
e) les antigènes hydrosolubles sont solubilisés dans une première solution comprenant Tris-HCl, dithiothréitol (DTT), un premier inhibiteur de protéase, et KCl.

5. Procédé selon l'une quelconque des revendications 1 à 4, dans lequel l'amphiphile dans la seconde solution est une sulfobétaïne.

6. Procédé selon la revendication 5, dans lequel la sulfobétaïne est sélectionnée parmi le groupe constitué de : 3-[N,N-diméthyl(3-myristoylaminopropyl)ammonio]propanesulfonate (amidosulfobétaïne-14 ; ASB-14) ; amidosulfobétaïne-16 (ASB-16) ; 4-n-octylbenzoylamido-propyl-diméthylammonio sulfobétaïne (ASB-C8Ø) ; sel interne de 3-(N,N-diméthyloctylammonio) propanesulfonate (SB3-8) ; N-décyl-N,N-diméthyl-3-ammonio-1-propanesulfonate (SB3-10), N-décyl-N,N-diméthyl-3-ammonio-1- propanesulfonate (SB3-12), N-tétradécyl-N,N-diméthyl-3-ammonio-1-propanesulfonate (SB3-14) ; 3-(N,N-diméthylpalmitylammonio) propanesulfonate (SB3-16) ; 3-(N,N-diméthyloctadécylammonio) propanesulfonate (SB3-18) ; 3-(1-pyridinio)-1-propanesulfonate (NDSB-201) ; 3-(benzyldiméthylammonio) propanesulfonate (NDSB-256) ; NDSB-211, NDSB-195, NDSB-221 ; 3-[(3-cholamidopropyl)diméthylammonio]-1-propanesulfonate (CHAPS), 3-[(3-cholamidopropyl)diméthylammonio]-2-hydroxy-1-propanesulfonate (CHAPSO), et mélanges de ceux-ci.

7. Procédé selon l'une quelconque des revendications 1 à 6, dans lequel les antigènes liposolubles sont solubilisés dans une solution comprenant Tris-HCl, dithiothréitol (DTT), un inhibiteur de protéase, KCl et ASB-14.

8. Procédé selon la revendication 7, comprenant en outre la repopulation de l'ECM avec des cellules vivantes.

9. Procédé selon l'une quelconque des revendications 1 à 8, dans lequel le tissu est :
a) tissu épithélial, tissu endothélial, tissu musculaire, ou tissu conjonctif ;
b) sélectionné parmi le groupe constitué de : tissu musculaire cardiaque, tissu musculaire strié ou squelettique, ou tissu musculaire lisse, cœur, péricarde, valvule cardiaque, vaisseau, conduit vasculaire, artère, veine, peau, derme, péricarde, dure-mère, sous-muqueuse intestinale, ligament, tendon, os, cartilage, urètre, vessie urinaire, rein, peau, poumon, foie, et cordon ombilical ;
c) tissu musculaire ;
d) tissu musculaire sélectionné parmi le groupe constitué de : tissu musculaire cardiaque, tissu musculaire strié ou squelettique et tissu musculaire lisse ; et/ou
e) une partie d'un organe intact.

10. Procédé selon l'une quelconque des revendications 1 à 9, dans lequel le tissu est un tissu intact, le procédé comprenant :
a) la solubilisation d'antigènes hydrosolubles dans le tissu par l'intermédiaire de la mise en contact du tissu avec la première solution comprenant Tris-HCl, dithiothréitol (DTT), l'inhibiteur de protéase, et KCl, dans lequel la première solution ne comprend pas d' amphiphile, de telle sorte que les antigènes hydrosolubles soient solubilisés dans la première solution ;
b) la séparation du tissu à partir des antigènes hydrosolubles solubilisés dans la première solution ;
c) la solubilisation d'antigènes liposolubles dans le tissu par l'intermédiaire de la mise en contact du tissu avec une seconde solution comprenant Tris-HCl, dithiothréitol (DTT), l'inhibiteur de protéase, KCl et ASB 14, de telle sorte que les antigènes liposolubles soient solubilisés dans la seconde solution ; et
d) la séparation du tissu à partir des antigènes liposolubles solubilisés dans la seconde solution ; ainsi éliminant des antigènes immunogènes à partir du tissu intact.

11. Procédé selon la revendication 1, dans lequel le tissu est un tissu musculaire, comprenant :
a) la relaxation du tissu musculaire dans une solution comprenant une molécule source d'énergie ;
b) la mise en contact du tissu musculaire avec un ou plusieurs agents de dépolymérisation d'actine, et/ou un ou plusieurs agents de dépolymérisation de microtubule ou de déstabilisation de microtubule ;
c) la mise en contact du tissu musculaire avec une solution de sel concentrée ;
d) la solubilisation d'antigènes hydrosolubles dans le tissu selon la revendication 1 ;
e) la séparation du tissu musculaire à partir des antigènes hydrosolubles solubilisés ;
f) la solubilisation d'antigènes liposolubles dans le tissu selon la revendication 1 ; et
g) la séparation du tissu musculaire à partir des antigènes liposolubles solubilisés ; ainsi éliminant des antigènes immunogènes à partir du tissu musculaire.

12. Procédé selon la revendication 11, dans lequel :
a) une ou plusieurs des étapes sont répétées ;
b) dans lequel les étapes sont réalisées dans un ordre sélectionné parmi le groupe constitué de :
i) a), b), c), d), e), f) et g) ;
ii) a), f), g), b), c), d) et e) ;
iii) a), d), e), b), c), f) et g) ;
iv) a), d), e), f), g), b) et c) ;
v) a), b), c), d), e), f), g), b), c), et e); et
vi) a), f), g), b), c), d), e), f), et g) ;
c) le tissu musculaire est un tissu musculaire cardiaque, un tissu musculaire strié ou squelettique, ou un tissu musculaire lisse ;
d) le procédé comprend l'étape de la relaxation du tissu musculaire avant la mise en contact du tissu avec un ou plusieurs agents de déstabilisation cytosquelettiques ; et/ou
e) des constituants sarcomères ne sont pas détectables dans le tissu musculaire.

13. Procédé selon la revendication 11, dans lequel la molécule source d'énergie est :
a) sélectionnée parmi le groupe constitué de : un 5'-triphosphate de nucléotide (NTP), adénosine, inosine, aspartate, glutamate, phosphate de créatine, un précurseur ou intermédiaire de cycle de Krebs, glucose, et dextrose ;
b) un 5'-triphosphate de nucléotide (NTP) sélectionné parmi le groupe constitué de : 5'-triphosphate d'adénosine (ATP), 5'-triphosphate d'inosine (ITP), de 5'-triphosphate de guanidine (GTP), 5'-triphosphate de cytidine (CTP), et 5'-triphosphate d'uridine (UTP) ; et/ou
c) un précurseur de 5'-triphosphate d'adénosine (ATP).

14. Procédé selon l'une quelconque des revendications 11 à 13, dans lequel la solution relaxante comprend en outre :
a) un agent chélateur à ions de calcium ;
b) un agent perméabilisateur ; et/ou
c) un inhibiteur de protéase et un agent antimicrobien.

15. Procédé selon l'une quelconque des revendications 11 à 14, dans lequel la solution de sel concentrée comprend :
a) un ou plusieurs sels dans une plage de concentration d'environ 0,5 M à environ 3,0 M; et/ou
b) un ou plusieurs sels d'halogénure.
